# EUROPEAN PATENT APPLICATION

(11) **EP 4 360 633 A2**
(43) Date of publication of application: **01.05.2024**
(21) Application number: 23220853.8
(22) Date of filing: 05.11.2015
(51) Int. Cl.: A61K 31/436

(54) **METHODS AND COMPOSITIONS RELATED TO THE USE OF LOW HLB SURFACTANTS IN THE PRODUCTION OF SYNTHETIC NANOCARRIERS COMPRISING A RAPALOG**

(30) Priority: 05.11.2014 US 201462075864 P; 05.11.2014 US 201462075866 P
(62) Divisional of application: 20204014.3
(71) Applicant: Selecta Biosciences, Inc., Watertown, MA 02472 (US)
(72) Inventor: O'NEIL, Conlin, Andover, 01810 (US); GRISET, Aaron, P., Somerville, 02144 (US); ALTREUTER, David, H., Wayland, 01778 (US)
(74) Representative: Keltie LLP

(57) **Abstract**

Disclosed are compositions and methods related to synthetic nanocarriers comprising a hydrophobic carrier material, a rapalog, and a non-ionic surfactant with a hydrophilic-lipophilic balance (HLB) value less than or equal to 10.

## Description

### RELATED APPLICATIONS

This application claims the benefit under 35 U.S.C. §119 of United States provisional application 62/075,864, filed November 5, 2014 and 62/075,866, filed November 5, 2014, the entire contents of each of which are incorporated herein by reference.

### FIELD OF THE INVENTION

This invention relates to synthetic nanocarriers, and related compositions and methods, comprising a hydrophobic carrier material, a rapalog, and a non-ionic surfactant with a hydrophilic-lipophilic balance (HLB) value less than or equal to 10.

### SUMMARY OF THE INVENTION

Provided herein are synthetic nanocarriers that can inhibit or reduce the immune response to an antigen, such as a co-administered antigen. The synthetic nanocarriers comprise a hydrophobic carrier material, a rapalog, and a non-ionic surfactant with a hydrophilic-lipophilic balance (HLB) value less than or equal to 10. It has been surprisingly discovered that the use of such a non-ionic surfactant in the formulation during synthetic nanocarrier formation, can result in synthetic nanocarriers with improved stability and performance.

In one aspect, a composition comprising synthetic nanocarriers comprising a hydrophobic carrier material, a rapalog, and a non-ionic surfactant with a hydrophilic-lipophilic balance (HLB) value less than or equal to 10, wherein the amount of non-ionic surfactant with HLB value less than or equal to 10 is ≥ 0.01 but ≤ 20 weight% non-ionic surfactant with a HLB value less than or equal to 10/hydrophobic carrier material is provided.

In one embodiment of any one of the compositions or methods provided herein, the weights are the recipe weights of the materials that are combined during the formulation of the synthetic nanocarriers. In one embodiment of any one of the compositions or methods provided herein, the weights are the weights of the materials in the resulting synthetic nanocarrier composition. In one embodiment of any one of the compositions or methods provided herein, the weights are the recipe weights of the materials that are combined during the formulation of the synthetic nanocarriers. In one embodiment of any one of the compositions or methods provided herein, the weights are the weights of the materials in the resulting synthetic nanocarrier composition.

In another aspect, a kit comprising any one of the compositions provided herein is provided. In one embodiment of any one of the kits provided, the composition is for use in any one of the methods provided herein. In one embodiment of any one of the kits provided, when the composition does not comprise antigen, the kit further comprises an antigen. In one embodiment of any one of the kits provided, the composition and antigen are contained in separate containers. In one embodiment of any one of the kits provided, the composition and antigen are contained in the same container. In one embodiment of any one of the kits provided, the kit further comprises instructions for use. In one embodiment of any one of the kits provided, the instructions for use include a description of any one of the methods provided herein.

In another aspect, a method comprising administering any one of the compositions provided herein to a subject is provided. In one embodiment of any one of the methods provided, when the composition does not comprise antigen, the method further comprises administering antigen to the subject. In one embodiment of any one of the methods provided, the antigen is comprised in different synthetic nanocarriers. In one embodiment of any one of the methods provided, the antigen is not coupled to any synthetic nanocarriers. In one embodiment of any one of the methods provided, the administering is by intradermal, intramuscular, intravenous, intraperitoneal or subcutaneous administration.

In another aspect, a method for producing synthetic nanocarriers comprising a non-ionic surfactant with HLB value less than or equal to 10 and a rapalog, comprising obtaining or providing a hydrophobic carrier material, obtaining or providing a non-ionic surfactant with HLB value less than or equal to 10, obtaining or providing a rapalog, and combining the hydrophobic carrier material, the non-ionic surfactant with HLB value less than or equal to 10 and the rapalog to form synthetic nanocarriers, wherein the amount of non-ionic surfactant with HLB value less than or equal to 10 in the synthetic nanocarriers is ≥ 0.01 but ≤ 20 weight% non-ionic surfactant with a HLB value less than or equal to 10/hydrophobic carrier material is provided. In one embodiment of any one of the methods for producing provided herein, the method further comprises dissolving the hydrophobic carrier material, non-ionic surfactant with HLB value less than or equal to 10 and rapalog in a solvent, obtaining or providing another surfactant, forming a first and then second O/W emulsion with the dissolved hydrophobic carrier material, non-ionic surfactant with HLB value less than or equal to 10 and rapalog, and other surfactant, mixing the first and second O/W emulsions, and allowing the solvent to evaporate. In one embodiment of any one of the methods for producing provided herein, the solvent is dichloromethane, ethyl acetate, chloroform, or propylene carbonate. In one embodiment of any one of the methods for producing provided herein, the method further comprises filtering the resulting composition. In one embodiment of any one of the methods for producing provided herein, the filtering comprises filtering through a 0.22 µm filter.

In another aspect, a composition produced by any one of the methods for producing provided herein is provided. The method for producing may be any one of the methods for producing provided, such as one of the methods illustrated in the **Examples.**

In one embodiment of any one of the compositions or methods provided herein, the synthetic nanocarrier composition is initially sterile filterable through a 0.22 µm filter.

In one embodiment of any one of the compositions or methods provided herein, the non-ionic surfactant with HLB value less than or equal to 10 is a non-ionic surfactant with HLB value less than 10. In one embodiment of any one of the compositions or methods provided herein, the non-ionic surfactant with HLB value less than or equal to 10 is a non-ionic surfactant with HLB value less than 9. In one embodiment of any one of the compositions or methods provided herein, the non-ionic surfactant with HLB value less than or equal to 10 is a non-ionic surfactant with HLB value less than 8. In one embodiment of any one of the compositions or methods provided herein, the non-ionic surfactant with HLB value less than or equal to 10 is a non-ionic surfactant with HLB value less than 7. In one embodiment of any one of the compositions or methods provided herein, the non-ionic surfactant with HLB value less than or equal to 10 is a non-ionic surfactant with HLB value less than 6. In one embodiment of any one of the compositions or methods provided herein, the non-ionic surfactant with HLB value less than or equal to 10 is a non-ionic surfactant with HLB value less than 5.

In one embodiment of any one of the compositions or methods provided herein, the non-ionic surfactant with HLB value less than or equal to 10 comprises a sorbitan ester, fatty alcohol, fatty acid ester, ethoxylated fatty alcohol, poloxamer, fatty acid, cholesterol, cholesterol derivative, or bile acid or salt. In one embodiment of any one of the compositions or methods provided herein, the non-ionic surfactant with HLB value less than or equal to 10 comprises SPAN 40, SPAN 20, oleyl alcohol, stearyl alcohol, isopropyl palmitate, glycerol monostearate, BRIJ 52, BRIJ 93, Pluronic P-123, Pluronic L-31, palmitic acid, dodecanoic acid, glyceryl tripalmitate or glyceryl trilinoleate. In one embodiment of any one of the compositions or methods provided herein, the non-ionic surfactant with HLB value less than or equal to 10 is SPAN 40.

In one embodiment of any one of the compositions or methods provided herein, the non-ionic surfactant with HLB value less than or equal to 10 is encapsulated in the synthetic nanocarriers, present on the surface of the synthetic nanocarriers, or both.

In one embodiment of any one of the compositions or methods provided herein, the amount of non-ionic surfactant with HLB value less than or equal to 10 is ≥ 0.1 but ≤ 15 weight% non-ionic surfactant with a HLB value less than or equal to 10/hydrophobic carrier material. In one embodiment of any one of the compositions or methods provided herein, the amount of non-ionic surfactant with HLB value less than or equal to 10 is ≥ 1 but ≤ 13 weight% non-ionic surfactant with a HLB value less than or equal to 10/hydrophobic carrier material. In one embodiment of any one of the compositions or methods provided herein, the amount of non-ionic surfactant with HLB value less than or equal to 10 is ≥ 1 but ≤ 9 weight% non-ionic surfactant with a HLB value less than or equal to 10/hydrophobic carrier material.

In one embodiment of any one of the compositions or methods provided herein, the hydrophobic carrier material comprises one or more hydrophobic polymers or lipids. In one embodiment of any one of the compositions or methods provided herein, the hydrophobic carrier material comprises one or more hydrophobic polymers, and wherein the one or more hydrophobic polymers comprise a polyester. In one embodiment of any one of the compositions or methods provided herein, the polyester comprises PLA, PLG, PLGA or polycaprolactone. In one embodiment of any one of the compositions or methods provided herein, the hydrophobic carrier material comprises or further comprises PLA-PEG, PLGA-PEG or PCL-PEG.

In one embodiment of any one of the compositions or methods provided herein, the amount of hydrophobic carrier material in the synthetic nanocarriers is 5-95 weight% hydrophobic carrier material/total solids. In one embodiment of any one of the compositions or methods provided herein, the amount of hydrophobic carrier material in the synthetic nanocarriers is 60-95 weight% hydrophobic carrier material/total solids.

In one embodiment of any one of the compositions or methods provided herein, the amount of rapalog is ≥ 6 but ≤ 50 weight% rapalog/hydrophobic carrier material. In one embodiment of any one of the compositions or methods provided herein, the amount of rapalog is ≥ 7 but ≤ 30 weight% rapalog/hydrophobic carrier material. In one embodiment of any one of the compositions or methods provided herein, the amount of rapalog is ≥ 8 but ≤ 24 weight% rapalog/hydrophobic carrier material.

In one embodiment of any one of the compositions or methods provided herein, the rapalog is encapsulated in the synthetic nanocarriers.

In one embodiment of any one of the compositions or methods provided herein, the rapalog is rapamycin.

In one embodiment of any one of the compositions or methods provided herein, the composition further comprises an antigen.

In one embodiment of any one of the compositions or methods provided herein, the antigen is admixed with the synthetic nanocarriers in the composition.

In one embodiment of any one of the compositions or methods provided herein, the mean of a particle size distribution obtained using dynamic light scattering of the synthetic nanocarriers is a diameter greater than 120nm. In one embodiment of any one of the compositions or methods provided herein, the diameter is greater than 150nm. In one embodiment of any one of the compositions or methods provided herein, the diameter is greater than 200nm. In one embodiment of any one of the compositions or methods provided herein, the diameter is greater than 250nm. In one embodiment of any one of the compositions or methods provided herein, the diameter is less than 300nm. In one embodiment of any one of the compositions or methods provided herein, the diameter is less than 250nm. In one embodiment of any one of the compositions or methods provided herein, the diameter is less than 200nm.

In one embodiment of any one of the compositions or methods provided herein, the composition further comprises a pharmaceutically acceptable carrier.

In one embodiment of any one of the compositions or methods provided, where the rapalog is rapamycin, the hydrophobic carrier material comprises PLA or PLGA and PLA-PEG, and the non-ionic surfactant with HLB value less than or equal to 10 is sorbitan monopalmitate, the diameter of the synthetic nanocarriers is as provided in any one of the compositions provided herein, such as any one of the compositions of the **Examples.** In one embodiment of any one of the compositions or methods provided, where the rapalog is rapamycin, the hydrophobic carrier material comprises PLA or PLGA and PLA-PEG, and the non-ionic surfactant with HLB value less than or equal to 10 is sorbitan monopalmitate, the diameter of the synthetic nanocarriers and/or the weight% of the rapamycin is as provided in any one of the compositions provided herein, such as any one of the compositions of the **Examples.** In one embodiment of any one of the compositions or methods provided, where the rapalog is rapamycin, the hydrophobic carrier material comprises PLA or PLGA and PLA-PEG, and the non-ionic surfactant with HLB value less than or equal to 10 is sorbitan monopalmitate, the diameter of the synthetic nanocarriers is as provided in any one of the compositions provided herein, such as any one of the compositions of the **Examples** and/or the weight% of the rapamycin is as provided in any one of the compositions provided herein, such as any one of the compositions of the **Examples** and/or the amount of the polymer(s) is as provided in any one of the compositions provided herein, such as any one of the compositions of the **Examples.** In one embodiment of any one of the compositions or methods provided, where the rapalog is rapamycin, the hydrophobic carrier material comprises PLA or PLGA and PLA-PEG, and the non-ionic surfactant with HLB value less than or equal to 10 is sorbitan monopalmitate, the diameter of the synthetic nanocarriers is as provided in any one of the compositions provided herein, such as any one of the compositions of the **Examples** and/or the weight% of the rapamycin is as provided in any one of the compositions provided herein, such as any one of the compositions of the **Examples** and/or the amount of the polymer(s) is as provided in any one of the compositions provided herein, such as any one of the compositions of the **Examples** and/or the amount of sorbitan monopalmitate is as provided in any one of the compositions provided herein, such as any one of the compositions of the **Examples.**

In another aspect, any one of the compositions provided herein, such as any one of the compositions of the **Examples** is provided.

In another aspect, a method of manufacturing any one of the compositions or kits provided herein is provided. In one embodiment, the method of manufacturing comprises the steps of any one of the methods provided herein, such as the steps of any one of the methods provided in the

### Examples.

In another aspect, a use of any one of the compositions or kits provided herein for the manufacture of a medicament for promoting immune tolerance in a subject is provided. In another embodiment of any one of the uses provided herein, the use is for achieving any one of the methods provided herein.

In another aspect, any one of the composition or kits provided herein may be for use in any one of the methods provided herein.

In another aspect, a method of manufacturing a medicament intended for promoting immune tolerance, is provided. In one embodiment, the medicament comprises any one of the compositions provided herein.

### BRIEF DESCRIPTION OF THE DRAWINGS

**Fig. 1** shows results demonstrating the ability of co-administered nanocarrier and KLH (keyhole limpet hemocyanin) with rapamycin (RAPA) to induce tolerance. The sera of the mice were analyzed for antibodies to KLH after each KLH challenge.
**Fig. 2** shows results demonstrating durable antibody titer reduction with nanocarriers with low HLB surfactants. The acronym "tSIP" refers to the nanocarriers as described.
**Fig. 3** shows results demonstrating synthetic nanocarrier+KLH efficacy compared to free rapamycin+KLH in mice. Anti-KLH EC50 at day 35 and 42 antibody titers (after 2 or 3 KLH alone challenges) for mice treated or not with synthetic nanocarrier+KLH (the symbols represent the geometric mean ± 95% CI). The acronym "NC" refers to the nanocarriers as described.
**Fig. 4** shows the treatment protocols for **Example 7.** The acronym "NC" refers to the nanocarriers as described.
**Fig. 5** shows synthetic nanocarrier+KLH antigen specificity in mice. Anti-OVA EC50 at Day 65 antibody titers for mice treated or not with synthetic nanocarrier+KLH (the bars represent the geometric mean ± 95% CI). The acronym "NC" refers to the nanocarriers as described.

### DETAILED DESCRIPTION OF THE INVENTION

Before describing the present invention in detail, it is to be understood that this invention is not limited to particularly exemplified materials or process parameters as such may, of course, vary. It is also to be understood that the terminology used herein is for the purpose of describing particular embodiments of the invention only, and is not intended to be limiting of the use of alternative terminology to describe the present invention.

All publications, patents and patent applications cited herein, whether supra or infra, are hereby incorporated by reference in their entirety for all purposes.

As used in this specification and the appended claims, the singular forms "a," "an" and "the" include plural referents unless the content clearly dictates otherwise. For example, reference to "a polymer" includes a mixture of two or more such molecules or a mixture of differing molecular weights of a single polymer species, reference to "a synthetic nanocarrier" includes a mixture of two or more such synthetic nanocarriers or a plurality of such synthetic nanocarriers, and the like.

As used herein, the term "comprise" or variations thereof such as "comprises" or "comprising" are to be read to indicate the inclusion of any recited integer (e.g. a feature, element, characteristic, property, method/process step or limitation) or group of integers (e.g. features, elements, characteristics, properties, method/process steps or limitations) but not the exclusion of any other integer or group of integers. Thus, as used herein, the term "comprising" is inclusive and does not exclude additional, unrecited integers or method/process steps.

In embodiments of any one of the compositions and methods provided herein, "comprising" may be replaced with "consisting essentially of" or "consisting of". The phrase "consisting essentially of' is used herein to require the specified integer(s) or steps as well as those which do not materially affect the character or function of the claimed invention. As used herein, the term "consisting" is used to indicate the presence of the recited integer (e.g. a feature, element, characteristic, property, method/process step or limitation) or group of integers (e.g. features, elements, characteristics, properties, method/process steps or limitations) alone.

### A. INTRODUCTION

It has been found that synthetic nanocarriers comprising hydrophobic carrier material in combination with a rapalog, such as rapamycin, may be initially difficult to sterile filter through a 0.22 µm filter. Use of such a filter in the production of synthetic nanocarriers is important, as the filters can remove bacteria to a desirable level and result in a composition that is more sterile, a beneficial feature for compositions used for *in vivo* administration. Surprisingly, due to the nature of the two hydrophobic components of such synthetic nanocarriers, it has been discovered that the incorporation of a non-ionic surfactant, such as sorbitan monopalmitate, in the synthetic nanocarriers can result in synthetic nanocarriers with improved filterability and, in some embodiments, improved rapalog loading, and thus efficacy when administered to a subject. Accordingly, it has been found that the use of non-ionic surfactants with a hydrophilic-lipophilic balance (HLB) value less than or equal to 10 can allow for a more efficacious load of a rapalog, preferably a hydrophobic one, while also improving initial sterile filterability.

As shown in the **Examples,** a number of such surfactants increased the throughput of synthetic nanocarrier formulations when initially passed through a 0.22 µm filter. Synthetic nanocarriers with such surfactants also were found to provide for higher loads of rapalog, such as hydrophobic rapalog, which were able to provide for durable antigen-specific tolerance in subjects. While the use of higher hydrophilic-lipophilic balance (HLB) surfactants is typical for the suspension or dissolution of hydrophobic agents into solvents, introduction of such agents into the oil-soluble phase during nanocarrier formulation, may not support the optimized manufacture of highly-loaded rapalog, such as hydrophobic rapalog, in nanocarriers. Rather, non-ionic surfactants with HLB ≤ 10, such as sorbitan monopalmitate (SPAN 40) (HLB 6.7), represent a class of surfactants that aid said optimization.

Accordingly, compositions of, and methods related thereto, synthetic nanocarriers comprising a hydrophobic carrier material, a rapalog, preferably a hydrophobic one, and a non-ionic surfactant with a hydrophilic-lipophilic balance (HLB) value less than or equal to 10 are provided. Such compositions can exhibit improved initial sterile filterability and, in some embodiments, improved rapalog, such as hydrophobic rapalog, loading and performance and can be used in methods to reduce unwanted antigen-specific immune system activation and/or promote antigen-specific tolerance.

The invention will now be described in more detail below.

### B. DEFINITIONS

"Administering" or "administration" or "administer" means providing a material to a subject in a manner that is pharmacologically useful. The term is intended to include causing to be administered in some embodiments. "Causing to be administered" means causing, urging, encouraging, aiding, inducing or directing, directly or indirectly, another party to administer the material.

"Admixed" refers to mixing one component, such as an antigen, with another, such as synthetic nanocarriers, in a composition. The components that are mixed are made or obtained separately and placed together. Accordingly, the components are not coupled to each other save for possible non-covalent interactions that may occur when placed together in a composition.

"Amount effective" in the context of a composition or dose for administration to a subject refers to an amount of the composition or dose that produces one or more desired responses in the subject, for example, the generation of an antigen-specific tolerogenic immune response. In some embodiments, the amount effective is a pharmacodynamically effective amount. Therefore, in some embodiments, an amount effective is any amount of a composition or dose provided herein that produces one or more of the desired therapeutic effects and/or immune responses as provided herein. This amount can be for *in vitro* or *in vivo* purposes. For *in vivo* purposes, the amount can be one that a clinician would believe may have a clinical benefit for a subject in need of antigen-specific immune tolerance. Any one of the compositions as provided herein can be in an amount effective.

Amounts effective can involve reducing the level of an undesired immune response, although in some embodiments, it involves preventing an undesired immune response altogether. Amounts effective can also involve delaying the occurrence of an undesired immune response. An amount that is effective can also be an amount that produces a desired therapeutic endpoint or a desired therapeutic result. In other embodiments, the amounts effective can involve enhancing the level of a desired response, such as a therapeutic endpoint or result. Amounts effective, preferably, result in a tolerogenic immune response in a subject to an antigen. The achievement of any of the foregoing can be monitored by routine methods.

Amounts effective will depend, of course, on the particular subject being treated; the severity of a condition, disease or disorder; the individual patient parameters including age, physical condition, size and weight; the duration of the treatment; the nature of concurrent therapy (if any); the specific route of administration and like factors within the knowledge and expertise of the health practitioner. These factors are well known to those of ordinary skill in the art and can be addressed with no more than routine experimentation. It is generally preferred that a maximum dose be used, that is, the highest safe dose according to sound medical judgment. It will be understood by those of ordinary skill in the art, however, that a patient may insist upon a lower dose or tolerable dose for medical reasons, psychological reasons or for virtually any other reason.

In general, doses of the components in the compositions of the invention refer to the amount of the components. Alternatively, the dose can be administered based on the number of synthetic nanocarriers that provide the desired amount.

"Antigen" means a B cell antigen or T cell antigen. "Type(s) of antigens" means molecules that share the same, or substantially the same, antigenic characteristics. In some embodiments, antigens may be proteins, polypeptides, peptides, lipoproteins, glycolipids, polynucleotides, polysaccharides or are contained or expressed in cells. In some embodiments, such as when the antigens are not well defined or characterized, the antigens may be contained within a cell or tissue preparation, cell debris, cell exosomes, conditioned media, etc.

"Antigen-specific" refers to any immune response that results from the presence of the antigen, or portion thereof, or that generates molecules that specifically recognize or bind the antigen. For example, where the immune response is antigen-specific antibody production, antibodies are produced that specifically bind the antigen. As another example, where the immune response is antigen-specific B cell or CD4+ T cell proliferation and/or activity, the proliferation and/or activity results from recognition of the antigen, or portion thereof, alone or in complex with MHC molecules, B cells, etc.

"Average", as used herein, refers to the arithmetic mean unless otherwise noted.

"Encapsulate" means to enclose at least a portion of a substance within a synthetic nanocarrier. In some embodiments, a substance is enclosed completely within a synthetic nanocarrier. In other embodiments, most or all of a substance that is encapsulated is not exposed to the local environment external to the synthetic nanocarrier. In other embodiments, no more than 50%, 40%, 30%, 20%, 10% or 5% (weight/weight) is exposed to the local environment. Encapsulation is distinct from absorption, which places most or all of a substance on a surface of a synthetic nanocarrier, and leaves the substance exposed to the local environment external to the synthetic nanocarrier. In embodiments of any one of the compositions or methods provided herein the rapalog and/or non-ionic surfactant with a hydrophilic-lipophilic balance (HLB) value less than or equal to 10 are encapsulated within the synthetic nanocarriers.

"Hydrophobic carrier material" refers to any pharmaceutically acceptable carrier that can deliver one or more molecules (e.g., a rapalog and a non-ionic surfactant with a HLB value less than or equal to 10) and that has hydrophobic characteristics. Such materials include materials that can form a synthetic nanocarrier or a portion thereof and that can include or be loaded with one or more molecules (e.g., a rapalog and a non-ionic surfactant with a HLB value less than or equal to 10). Generally, carrier materials can allow for delivery of one or more molecules (e.g., a rapalog and a non-ionic surfactant with a HLB value less than or equal to 10) to a target site or target cell, controlled-release of the one or more molecules, and other desired activities. Examples of hydrophobic carrier materials include, without limitation, materials that can be used to form synthetic nanocarriers and that comprise hydrophobic components, such as hydrophobic polymers, such as polyesters, or lipids. "Hydrophobic" refers to a material that does not substantially participate in hydrogen bonding to water. Such materials are generally non-polar, primarily non-polar, or neutral in charge. A carrier material suitable for the compositions described herein may be selected based on it exhibiting hydrophobicity at some level. Hydrophobic carrier materials, therefore, are those that are hydrophobic overall and may be completely comprised of hydrophobic components, such as hydrophobic polymers or lipids. In some embodiments, however, the hydrophobic carrier materials are hydrophobic overall and comprise hydrophobic components, such as hydrophobic polymers or lipids, in combination with non-hydrophobic components.

"Initially sterile filterable" refers to a composition of synthetic nanocarriers that has not previously been filtered but can be filtered through a filter, such as a 0.22 µm filter, with a throughput of at least 50 grams nanocarrier/m² of filter membrane surface area. In some embodiments of any one of the compositions or methods provided herein, the throughput is determined by taking a 9 mL volume of synthetic nanocarrier suspension and placing it in a 10 mL syringe with any one of the filters as provided herein. The synthetic nanocarrier suspension is then pushed through the filter until no further suspension materials pass through the filter. The throughput can then be calculated based on the material that was pushed through the filter and the remaining suspension material in the syringe. In some embodiments of any one of the compositions or methods provided herein, the initially sterile filterable composition is non-sterile and/or not suitable for *in vivo* administration (i.e., not substantially pure and comprising soluble components that are less than desirable for administration *in vivo*). In other embodiments of any one of the compositions or methods provided herein, the initially sterile filterable composition comprises synthetic nanocarriers that have been produced but have not been further processed to produce a clinical grade material. In some embodiments of any one of the compositions or methods provided herein, the initially sterile filterable composition has not previously been filtered but can be filtered through a filter, such as a 0.22 µm filter, with a throughput of at least 60, 70, 80, 90, 100, 120, 130, 140, 160, 200, 250, 300, 350, 500, 750, 1000 or 1500 grams nanocarrier/m² of a filter membrane surface area. The 0.22 µm filter can be any filter with a 0.22 µm pore size. Such filters can be made of a variety of materials, such as polyethylene sulfone, polyvinylidene fluoride, mixed cellulose esters, solvent free cellulose acetate, regenerated cellulose, nylon, etc. Specific examples of filters include Millipore SLGPM33R, Millipore SLGVM33RS, Millipore SLGSM33SS, Sartorius 16534, Sartorius 17764, Sartorius 17845, etc.

"Maximum dimension of a synthetic nanocarrier" means the largest dimension of a nanocarrier measured along any axis of the synthetic nanocarrier. "Minimum dimension of a synthetic nanocarrier" means the smallest dimension of a synthetic nanocarrier measured along any axis of the synthetic nanocarrier. For example, for a spheroidal synthetic nanocarrier, the maximum and minimum dimension of a synthetic nanocarrier would be substantially identical, and would be the size of its diameter. Similarly, for a cuboidal synthetic nanocarrier, the minimum dimension of a synthetic nanocarrier would be the smallest of its height, width or length, while the maximum dimension of a synthetic nanocarrier would be the largest of its height, width or length. In an embodiment, a minimum dimension of at least 75%, preferably at least 80%, more preferably at least 90%, of the synthetic nanocarriers in a sample, based on the total number of synthetic nanocarriers in the sample, is equal to or greater than 100 nm. In an embodiment, a maximum dimension of at least 75%, preferably at least 80%, more preferably at least 90%, of the synthetic nanocarriers in a sample, based on the total number of synthetic nanocarriers in the sample, is equal to or less than 5 µm. Preferably, a minimum dimension of at least 75%, preferably at least 80%, more preferably at least 90%, of the synthetic nanocarriers in a sample, based on the total number of synthetic nanocarriers in the sample, is greater than 110 nm, more preferably greater than 120 nm, more preferably greater than 130 nm, and more preferably still greater than 150 nm. Aspects ratios of the maximum and minimum dimensions of synthetic nanocarriers may vary depending on the embodiment. For instance, aspect ratios of the maximum to minimum dimensions of the synthetic nanocarriers may vary from 1:1 to 1,000,000:1, preferably from 1:1 to 100,000:1, more preferably from 1:1 to 10,000:1, more preferably from 1:1 to 1000: 1, still more preferably from 1:1 to 100:1, and yet more preferably from 1:1 to 10:1.

Preferably, a maximum dimension of at least 75%, preferably at least 80%, more preferably at least 90%, of the synthetic nanocarriers in a sample, based on the total number of synthetic nanocarriers in the sample is equal to or less than 3 µm, more preferably equal to or less than 2 µm, more preferably equal to or less than 1 µm, more preferably equal to or less than 800 nm, more preferably equal to or less than 600 nm, and more preferably still equal to or less than 500 nm. In preferred embodiments, a minimum dimension of at least 75%, preferably at least 80%, more preferably at least 90%, of the synthetic nanocarriers in a sample, based on the total number of synthetic nanocarriers in the sample, is equal to or greater than 100 nm, more preferably equal to or greater than 120 nm, more preferably equal to or greater than 130 nm, more preferably equal to or greater than 140 nm, and more preferably still equal to or greater than 150 nm. Measurement of synthetic nanocarrier dimensions (*e.g*., effective diameter) may be obtained, in some embodiments, by suspending the synthetic nanocarriers in a liquid (usually aqueous) media and using dynamic light scattering (DLS) (*e.g*., using a Brookhaven ZetaPALS instrument). For example, a suspension of synthetic nanocarriers can be diluted from an aqueous buffer into purified water to achieve a final synthetic nanocarrier suspension concentration of approximately 0.01 to 0.5 mg/mL. The diluted suspension may be prepared directly inside, or transferred to, a suitable cuvette for DLS analysis. The cuvette may then be placed in the DLS, allowed to equilibrate to the controlled temperature, and then scanned for sufficient time to acquire a stable and reproducible distribution based on appropriate inputs for viscosity of the medium and refractive indicies of the sample. The effective diameter, or mean of the distribution, is then reported. Determining the effective sizes of high aspect ratio, or non-spheroidal, synthetic nanocarriers may require augmentative techniques, such as electron microscopy, to obtain more accurate measurements. "Dimension" or "size" or "diameter" of synthetic nanocarriers means the mean of a particle size distribution, for example, obtained using dynamic light scattering.

"Non-ionic surfactant with a HLB value less than or equal to 10", or "low HLB surfactant", as used herein, refers to a non-ionic amphiphilic molecule that has a structure comprising at least one hydrophobic tail and a hydrophilic head or that has hydrophobic groups or regions and hydrophilic groups or regions. The tail portion of surfactants generally consists of a hydrocarbon chain. Surfactants can be classified based on the charge characteristics of the hydrophilic head portion or groups or regions. As used herein, "HLB" refers to the hydrophilic-lipophilic balance or hydrophile-lipophile balance of a surfactant and is a measure of the hydrophilic or lipophilic nature of a surfactant.

The HLB of any one of surfactants provided herein may be calculated using the Griffin's method or the Davie's method. For example, using the Griffin's method, the HLB of a surfactant is the product of 20 multiplied by the molecular mass of the hydrophilic portion of the surfactant divided by the molecular mass of the entire surfactant. The HLB value is on a scale from 0 to 20, with 0 corresponding to a completely hydrophobic (lipophilic) molecule, and 20 corresponding to a completely hydrophilic (lipophobic) molecule. In some embodiments, the HLB of the surfactant of any one of the compositions or methods provided herein is 0, 1, 2, 3, 4, 5, 6, 7, 8, 9, or 10 (e.g., as determined by Griffin's or Davie's method). Examples of such surfactants for use in any one of the compositions and methods provided herein include, without limitation, sorbitan esters, such as SPAN 40, SPAN 20; fatty alcohols, such as oleyl alcohol, stearyl alcohol; fatty acid esters, such as isopropyl palmitate, glycerol monostearate; ethoxylated fatty alcohols, such as BRIJ 52, BRIJ 93; poloxamers, such as Pluronic P-123, Pluronic L-31; fatty acids, such as palmitic acid, dodecanoic acid; triglycerides, such as glyceryl tripalmitate, glyceryl trilinoleate; cholesterol; cholesterol derivatives, such as sodium cholesteryl sulfate, cholesteryl dodecanoate; and bile salts or acids, such as lithocholic acid, sodium lithocholate. Further examples of such surfactants include sorbitan monostearate (SPAN 60), sorbitan tristearate (SPAN 65), sorbitan monooleate (SPAN 80), sorbitan sesquioleate (SPAN 83), sorbitan trioleate (SPAN 85), sorbitan sesquioleate (Arlacel 83), sorbitan dipalmitate, mono and diglycerides of fatty acids, polyoxyethylene sorbitan trioleate (Tween 85), polyoxyethylene sorbitan hexaoleate (G 1086), sorbitan monoisostearate (Montane 70), polyoxyethylene alcohols, polyoxyethylene glycol alkyl ethers, polyoxyethylene (2) oleyl ether (BRIJ 93), polyoxyethylene cetyl ether (BRIJ 52), polyethylene glycol dodecyl ether (BRIJ L4); 1-monotetradecanoyl-rac-glycerol; glyceryl monostearate; glycerol monopalmitate; ethylenediamine tetradkis tetrol (Tetronic 90R4, Tetronic 701), polyoxyethylene (5) nonylphenylether (IGEPAL CA-520), MERPOL A surfactant, MERPOL SE surfactant, and poly(ethylene glycol) sorbitol hexaoleate. Further examples would also be apparent to one of ordinary skill in the art.

"Pharmaceutically acceptable excipient" or "pharmaceutically acceptable carrier" means a pharmacologically inactive material used together with a pharmacologically active material to formulate the compositions. Pharmaceutically acceptable excipients comprise a variety of materials known in the art, including but not limited to saccharides (such as glucose, lactose, and the like), preservatives such as antimicrobial agents, reconstitution aids, colorants, saline (such as phosphate buffered saline), and buffers.

"Providing" means an action or set of actions that an individual performs that supplies a needed item or set of items or methods for the practice of the present invention. The action or set of actions may be taken either directly oneself or indirectly.

"Rapalog" refers to rapamycin and molecules that are structurally related to (an analog) of rapamycin (sirolimus), and are preferably hydrophobic. Examples of rapalogs include, without limitation, temsirolimus (CCI-779), deforolimus, everolimus (RAD001), ridaforolimus (AP-23573), zotarolimus (ABT-578). Additional examples of rapalogs may be found, for example, in WO Publication WO 1998/002441 and U.S. Patent No. 8,455,510, the disclosure of such rapalogs are incorporated herein by reference in its entirety.

"Solvent" refers to a substance that can dissolve a solute, such as any one or more of the components of the synthetic nanocarriers as provided herein. In some embodiments, the solvents are those that are useful in the formation of synthetic nanocarriers, such as in an emulsion process (e.g., a double emulsion process). Examples of such solvents include dichloromethane, ethyl acetate, chloroform, and propylene carbonate. Examples also include solvent mixtures that are a combination of a low aqueous solubility organic solvent and a water miscible solvent, such as acetone, ethanol, dimethylsulfoxide, dimethylformamide, formamide, etc. Further examples will be known to one of ordinary skill in the art.

"Subject" means animals, including warm blooded mammals such as humans and primates; avians; domestic household or farm animals such as cats, dogs, sheep, goats, cattle, horses and pigs; laboratory animals such as mice, rats and guinea pigs; fish; reptiles; zoo and wild animals; and the like.

"Surfactant" refers to a compound that can lower the surface tension between two liquids or between a liquid and a solid. Surfactants may act as detergents, wetting agents, emulsifiers, foaming agents, and dispersants and can be used in the formation of synthetic nanocarriers as provided herein. In some embodiments, the surfactants are non-ionic surfactants with a HLB value less than or equal to 10.

"Synthetic nanocarrier(s)" means a discrete object that is not found in nature, and that possesses at least one dimension that is less than or equal to 5 microns in size. As provided herein the synthetic nanocarriers comprise a hydrophobic carrier material. Accordingly, a synthetic nanocarrier can be, but is not limited to, synthetic nanocarriers comprising hydrophobic polymeric nanoparticles as well as lipid-based nanoparticles. Synthetic nanocarriers may be a variety of different shapes, including but not limited to spheroidal, cuboidal, pyramidal, oblong, cylindrical, toroidal, and the like. Synthetic nanocarriers according to the invention comprise one or more surfaces. In embodiments, synthetic nanocarriers may possess an aspect ratio greater than 1:1, 1:1.2, 1:1.5, 1:2, 1:3, 1:5, 1:7, or greater than 1:10.

Synthetic nanocarriers according to the invention that have a minimum dimension of equal to or less than about 100 nm, preferably equal to or less than 100 nm, do not comprise a surface with hydroxyl groups that activate complement or alternatively comprise a surface that consists essentially of moieties that are not hydroxyl groups that activate complement. In a preferred embodiment, synthetic nanocarriers according to the invention that have a minimum dimension of equal to or less than about 100 nm, preferably equal to or less than 100 nm, do not comprise a surface that substantially activates complement or alternatively comprise a surface that consists essentially of moieties that do not substantially activate complement. In a more preferred embodiment, synthetic nanocarriers according to the invention that have a minimum dimension of equal to or less than about 100 nm, preferably equal to or less than 100 nm, do not comprise a surface that activates complement or alternatively comprise a surface that consists essentially of moieties that do not activate complement.

"Total solids" refers to the total weight of all components contained in a composition or suspension of synthetic nanocarriers. In some embodiments of any one of the compositions or methods provided herein, the amount of total solids is determined as the total dry-nanocarrier mass per mL of suspension. This can be determined by a gravimetric method.

"Weight%" refers to the ratio of one weight to another weight times 100. For example, the weight% can be the ratio of the weight of one component to another times 100 or the ratio of the weight of one component to a total weight of more than one component times 100. Generally, the weight% is measured as an average across a population of synthetic nanocarriers or an average across the synthetic nanocarriers in a composition or suspension.

### C. COMPOSITIONS AND RELATED METHODS

Provided herein are synthetic nanocarrier compositions with improved initial sterile filterability and, in some embodiments, improved rapalog loading and, thus, efficacy when administered to a subject. Such synthetic nanocarriers comprise a hydrophobic carrier material as well as a rapalog, which is preferably hydrophobic. Surprisingly, it was found that a certain class of surfactants, non-ionic surfactants with a hydrophilic-lipophilic balance (HLB) value less than or equal to 10, were able to allow for improved initial sterile filterability and, in some embodiments, more efficacious loads of rapalogs in synthetic nanocarriers. As shown in the **Examples,** increased throughput of synthetic nanocarrier compositions, when initially passed through a 0.22 µm filter, was found when surfactants, like SPAN 40, were incorporated in the synthetic nanocarrier compositions. As also shown in the **Examples,** a number of such synthetic nanocarriers formulated with a non-ionic surfactant with a hydrophilic-lipophilic balance (HLB) value less than or equal to 10 were also able to provide for durable antigen-specific tolerance in subjects.

Optimized amounts of the non-ionic surfactant with HLB value less than or equal to 10 in the synthetic nanocarriers as provided herein have also been discovered. In some embodiments of any one of the compositions or methods provided herein, the amount of the non-ionic surfactant with HLB value less than or equal to 10 in the synthetic nanocarriers is ≥ 0.01 but ≤ 20 weight% non-ionic surfactant with a HLB value less than or equal to 10/hydrophobic carrier material. In some embodiments of any one of the compositions or methods provided herein, the amount of the non-ionic surfactant with HLB value less than or equal to 10 in the synthetic nanocarriers is ≥ 0.1 but ≤ 15, ≥ 0.5 but ≤ 13, ≥ 1 but ≤ 9 or 10 weight% non-ionic surfactant with a HLB value less than or equal to 10/hydrophobic carrier material. In other embodiments of any one of the compositions or methods provided herein, the amount of the non-ionic surfactant with HLB value less than or equal to 10 in the synthetic nanocarriers is ≥ 0.01 but ≤ 17, ≥ 0.01 but ≤ 15, ≥ 0.01 but ≤ 13, ≥ 0.01 but ≤ 12, ≥ 0.01 but ≤11, ≥ 0.01 but ≤ 10, ≥ 0.01 but ≤ 9, ≥ 0.01 but ≤ 8 ≥ 0.01 but ≤ 7, ≥ 0.01 but ≤ 6, ≥ 0.01 but ≤ 5, etc. weight% non-ionic surfactant with a HLB value less than or equal to 10/hydrophobic carrier material. In still other embodiments of any one of the compositions or methods provided herein, the amount of the non-ionic surfactant with HLB value less than or equal to 10 in the synthetic nanocarriers is ≥ 0.1 but ≤ 15, ≥ 0.1 but ≤ 14, ≥ 0.1 but ≤ 13, ≥ 0.1 but ≤ 12, ≥ 0.1 but ≤ 11, ≥ 0.1 but ≤ 10,≥ 0.1 but ≤ 9, ≥ 0.1 but ≤ 8, ≥ 0.1 but ≤ 7, ≥ 0.1 but ≤ 6, ≥ 0.1 but ≤ 5, etc. weight% non-ionic surfactant with a HLB value less than or equal to 10/hydrophobic carrier material. In still other embodiments of any one of the compositions or methods provided herein, the amount of the non-ionic surfactant with HLB value less than or equal to 10 in the synthetic nanocarriers is ≥ 0.5 but ≤ 15, ≥ 0.5 but ≤ 14, ≥ 0.5 but ≤ 13, ≥ 0.5 but ≤ 1 2, ≥ 0.5 but ≤ 11, ≥ 0.5 but ≤ 10, ≥ 0.5 but ≤ 9, ≥ 0.5 but ≤ 8, ≥ 0.5 but ≤ 7, ≥ 0.5 but ≤ 6, ≥ 0.5 but ≤ 5, etc. weight% non-ionic surfactant with a HLB value less than or equal to 10/hydrophobic carrier material. In still other embodiments of any one of the compositions or methods provided herein, the amount of the non-ionic surfactant with HLB value less than or equal to 10 in the synthetic nanocarriers is ≥ 1 but ≤ 9, ≥ 1 but ≤ 8, ≥ 1 but ≤ 7, ≥ 1 but ≤ 6, ≥ 1 but ≤ 5, etc. weight% non-ionic surfactant with a HLB value less than or equal to 10/hydrophobic carrier material. In still other embodiments of any one of the compositions or methods provided herein, the amount of the non-ionic surfactant with HLB value less than or equal to 10 in the synthetic nanocarriers is ≥ 5 but ≤ 15, ≥ 5 but ≤ 14, ≥ 5 but ≤ 13, ≥ 5 but ≤ 12, ≥ 5 but ≤ 11, ≥ 5 but ≤ 10, ≥ 5 but ≤ 9, ≥ 5 but ≤ 8, ≥ 5 but ≤ 7, ≥ 5 but ≤ 6, etc. weight% non-ionic surfactant with a HLB value less than or equal to 10/hydrophobic carrier material. In some embodiments of any one of the compositions or methods provided herein, the amount of the non-ionic surfactant with HLB value less than or equal to 10 in the synthetic nanocarriers is 1, 2, 3, 4, 5, 6, 7, 8, 9, 10, 11, 12, 13, 14, 15, 16, 17, 18, 19 or 20 weight% non-ionic surfactant with a HLB value less than or equal to 10/hydrophobic carrier material. Any one of the HLB values provided herein may be determined using Griffin's or Davie's method.

Optimized amounts of the hydrophobic carrier material in the synthetic nanocarrier compositions have also been determined. Preferably, in some embodiments of any one of the compositions or methods provided herein, the amount of hydrophobic carrier material in the synthetic nanocarrier composition is 5-95 weight% hydrophobic carrier material/total solids. In other embodiments of any one of the compositions or methods provided herein, the amount of hydrophobic carrier material in the synthetic nanocarriers is 10-95, 15-90, 20-90, 25-90, 30-80, 30-70, 30-60, 30-50, etc. weight% hydrophobic carrier material/total solids. In still other embodiments of any one of the compositions or methods provided herein, the amount of hydrophobic carrier materials in the synthetic nanocarriers is 5, 10, 15, 20, 25, 30, 35, 40, 45, 50, 55, 60, 65, 70, 75, 80, 85, 90 or 95 weight% hydrophobic carrier material/total solids.

Further, the amount of rapalog, such as rapamycin, in the synthetic nanocarriers can also be optimized. Preferably, such amounts can result in efficacious results (e.g., durable antigen-specific tolerance) when the synthetic nanocarriers are administered to a subject. In some embodiments of any one of the compositions or methods provided herein, the synthetic nanocarriers comprise ≥ 6 but ≤ 50 weight% rapalog/hydrophobic carrier material. In some embodiments of any one of the compositions or methods provided herein, the synthetic nanocarriers comprise ≥ 6 but ≤ 45, ≥ 6 but ≤ 40, ≥ 6 but ≤ 35, ≥ 6 but ≤ 30, ≥ 6 but ≤ 25, ≥ 6 but ≤ 20, ≥ 6 but ≤ 15 weight% rapalog/hydrophobic carrier material. In other embodiments of any one of the compositions or methods provided herein, the synthetic nanocarriers comprise ≥ 7 but ≤ 45, ≥ 7 but ≤ 40, ≥ 7 but ≤ 35, ≥ 7 but ≤ 30, ≥ 7 but ≤ 25, ≥ 7 but ≤ 20, ≥ 7 but ≤ 15 weight% rapalog/hydrophobic carrier material. In still other embodiments of any one of the compositions or methods provided herein, the synthetic nanocarriers comprise ≥ 8 but ≤ 24 weight% rapalog/hydrophobic carrier material. In some embodiments of any one of the compositions or methods provided herein, the synthetic nanocarriers comprise 6, 7, 8, 9, 10, 12, 15, 17, 20, 22, 25, 27, 30, 35, 45 or more weight% rapalog/hydrophobic carrier material.

The amounts of components or materials as recited herein for any one of the compositions or methods provided herein can be determined using methods known to those of ordinary skill in the art or otherwise provided herein. For example, amounts of the non-ionic surfactant with a HLB value less than or equal to 10 can be measured by extraction followed by quantitation by an HPLC method. Amounts of hydrophobic carrier material can be determined using HPLC. The determination of such an amount may, in some embodiments, follow the use of proton NMR or other orthogonal methods, such as MALDI-MS, etc. to determine the identity of a hydrophobic carrier material. Similar methods can be used to determine the amounts of rapalog in any one of the compositions provided herein. In some embodiments, the amount of rapalog is determined using HPLC. Further examples of methods for determining amounts of components or materials are as provided elsewhere herein, such as in the **Examples.** For any one of the compositions or methods provided herein the amounts of the components or materials can also be determined based on the recipe weights of a nanocarrier formulation. Accordingly, in some embodiments of any one of the compositions or methods provided herein, the amounts of any one of the components provided herein are those of the components in an aqueous phase during formulation of the synthetic nanocarriers. In some embodiments of any one of the compositions or methods provided herein, the amounts of any one of the components are those of the components in a synthetic nanocarrier composition that is produced and the result of a formulation process.

The synthetic nanocarriers as provided herein comprise hydrophobic carrier materials, such as hydrophobic polymers or lipids. Therefore, in some embodiments, the synthetic nanocarriers provided herein comprise one or more lipids. In some embodiments, a synthetic nanocarrier may comprise a lipid bilayer. In some embodiments, a synthetic nanocarrier may comprise a lipid monolayer. In some embodiments, a synthetic nanocarrier may comprise a core comprising a polymeric matrix surrounded by a lipid layer *(e.g.,* lipid bilayer, lipid monolayer, etc.). Further hydrophobic carrier materials include lipids (synthetic and natural), lipid-polymer conjugates, lipid-protein conjugates, and crosslinkable-oils, waxes, fats, etc. Further examples of lipid materials for use as hydrophobic carrier materials as provided herein can be found, for example, in PCT Publication No. WO2000/006120 and WO2013/056132, the disclosures of such materials being incorporated herein by reference in their entirety.

Accordingly, in some embodiments the synthetic nanocarriers provided herein can be liposomes. Liposomes can be produced by standard methods such as those reported by Kim et al. (1983, Biochim. Biophys. Acta 728, 339-348); Liu et al. (1992, Biochim. Biophys. Acta 1104, 95-101); Lee et al. (1992, Biochim. Biophys. Acta. 1103, 185-197), Brey et al. (U.S. Pat. Appl. Pub. 20020041861), Hass et al. (U.S. Pat. Appl. Pub. 20050232984), Kisak et al. (U.S. Pat. Appl. Pub. 20050260260) and Smyth-Templeton et al. (U.S. Pat. Appl. Pub. 20060204566), the disclosure of such liposomes and methods for their production are incorporated herein by reference in their entirety.

The hydrophobic carrier material as provided herein comprises one or more hydrophobic polymers or units thereof. However, in some embodiments, while the hydrophobic carrier material is hydrophobic overall, the hydrophobic carrier material may also comprise polymers or units thereof that are not hydrophobic.

Accordingly, the hydrophobic carrier material as provided herein may comprise polyesters, such as hydrophobic polyesters. Polyesters include copolymers comprising lactic acid and glycolic acid units, such as poly(lactic acid-co-glycolic acid) and poly(lactide-co-glycolide), collectively referred to herein as "PLGA"; and homopolymers comprising glycolic acid units, referred to herein as "PGA," and lactic acid units, such as poly-L-lactic acid, poly-D-lactic acid, poly-D,L-lactic acid, poly-L-lactide, poly-D-lactide, and poly-D,L-lactide, collectively referred to herein as "PLA." In some embodiments, exemplary polyesters include, for example, polyhydroxyacids; PEG copolymers and copolymers of lactide and glycolide (e.g., PLA-PEG copolymers, PGA-PEG copolymers, PLGA-PEG copolymers, and derivatives thereof. In some embodiments, polyesters include, for example, poly(caprolactone), poly(caprolactone)-PEG copolymers, poly(L-lactide-co-L-lysine), poly(serine ester), poly(4-hydroxy-L-proline ester), poly[α-(4-aminobutyl)-L-glycolic acid], and derivatives thereof.

In some embodiments, a polymer of the hydrophobic carrier material may be PLGA. PLGA is a biocompatible and biodegradable co-polymer of lactic acid and glycolic acid, and various forms of PLGA are characterized by the ratio of lactic acid:glycolic acid. Lactic acid can be L-lactic acid, D-lactic acid, or D,L-lactic acid. The degradation rate of PLGA can be adjusted by altering the lactic acid:glycolic acid ratio. In some embodiments, PLGA to be used in accordance with the present invention is characterized by a lactic acid:glycolic acid ratio of approximately 85:15, approximately 75:25, approximately 60:40, approximately 50:50, approximately 40:60, approximately 25:75, or approximately 15:85.

Hydrophobic carrier materials as provided herein may comprise one or more polymers that are a non-methoxy-terminated, pluronic polymer, or a unit thereof. "Non-methoxy-terminated polymer" means a polymer that has at least one terminus that ends with a moiety other than methoxy. In some embodiments, the polymer has at least two termini that ends with a moiety other than methoxy. In other embodiments, the polymer has no termini that ends with methoxy. "Non-methoxy-terminated, pluronic polymer" means a polymer other than a linear pluronic polymer with methoxy at both termini.

Hydrophobic carrier materials may comprise, in some embodiments, polyhydroxyalkanoates, polyamides, polyethers, polyolefins, polyacrylates, polycarbonates, polystyrene, silicones, fluoropolymers, or a unit thereof. Further examples of polymers that may be comprised in the hydrophobic carrier materials provided herein include polycarbonate, polyamide, or polyether, or unit thereof. In other embodiments, the polymers of the hydrophobic carrier material may comprise poly(ethylene glycol) (PEG), polypropylene glycol, or unit thereof.

In some embodiments, it is preferred that the hydrophobic carrier material comprises polymer that is biodegradable. Therefore, in such embodiments, the polymers of the hydrophobic carrier materials may include a polyether, such as poly(ethylene glycol) or polypropylene glycol or unit thereof. Additionally, the polymer may comprise a block-co-polymer of a polyether and a biodegradable polymer such that the polymer is biodegradable. In other embodiments, the polymer does not solely comprise a polyether or unit thereof, such as poly(ethylene glycol) or polypropylene glycol or unit thereof.

Other examples of polymers suitable for use in the present invention include, but are not limited to polyethylenes, polycarbonates (e.g. poly(1,3-dioxan-2one)), polyanhydrides (e.g. poly(sebacic anhydride)), polypropylfumerates, polyamides (e.g. polycaprolactam), polyacetals, polyethers, polyesters (e.g., polylactide, polyglycolide, polylactide-co-glycolide, polycaprolactone, polyhydroxyacid (e.g. poly(β-hydroxyalkanoate))), poly(orthoesters), polycyanoacrylates, polyvinyl alcohols, polyurethanes, polyphosphazenes, polyacrylates, polymethacrylates, polyureas, polystyrenes, and polyamines, polylysine, polylysine-PEG copolymers, and poly(ethyleneimine), poly(ethylene imine)-PEG copolymers.

Still other examples of polymers that may be included in a hydrophobic carrier material include acrylic polymers, for example, acrylic acid and methacrylic acid copolymers, methyl methacrylate copolymers, ethoxyethyl methacrylates, cyanoethyl methacrylate, aminoalkyl methacrylate copolymer, poly(acrylic acid), poly(methacrylic acid), methacrylic acid alkylamide copolymer, poly(methyl methacrylate), poly(methacrylic acid anhydride), methyl methacrylate, polymethacrylate, poly(methyl methacrylate) copolymer, polyacrylamide, aminoalkyl methacrylate copolymer, glycidyl methacrylate copolymers, polycyanoacrylates, and combinations comprising one or more of the foregoing polymers.

In some embodiments, the polymers of the hydrophobic carrier material can associate to form a polymeric matrix. A wide variety of polymers and methods for forming polymeric matrices therefrom are known conventionally. In some embodiments, a synthetic nanocarrier comprising a hydrophobic polymeric matrix generates a hydrophobic environment within the synthetic nanocarrier.

In some embodiments, polymers may be modified with one or more moieties and/or functional groups. A variety of moieties or functional groups can be used in accordance with the present invention. In some embodiments, polymers may be modified with polyethylene glycol (PEG), with a carbohydrate, and/or with acyclic polyacetals derived from polysaccharides (Papisov, 2001, ACS Symposium Series, 786:301). Certain embodiments may be made using the general teachings of US Patent No. 5543158 to Gref et al., or WO publication WO2009/051837 by Von Andrian et al.

In some embodiments, polymers may be modified with a lipid or fatty acid group. In some embodiments, a fatty acid group may be one or more of butyric, caproic, caprylic, capric, lauric, myristic, palmitic, stearic, arachidic, behenic, or lignoceric acid. In some embodiments, a fatty acid group may be one or more of palmitoleic, oleic, vaccenic, linoleic, alpha-linoleic, gammalinoleic, arachidonic, gadoleic, arachidonic, eicosapentaenoic, docosahexaenoic, or erucic acid.

In some embodiments, it is preferred that the polymer is biodegradable. In some embodiments, polymers in accordance with the present invention include polymers which have been approved for use in humans by the U.S. Food and Drug Administration (FDA) under 21 C.F.R. § 177.2600.

Polymers may be natural or unnatural (synthetic) polymers. Polymers may be homopolymers or copolymers comprising two or more monomers. In terms of sequence, copolymers may be random, block, or comprise a combination of random and block sequences. Typically, polymers in accordance with the present invention are organic polymers.

In some embodiments, polymers can be linear or branched polymers. In some embodiments, polymers can be dendrimers. In some embodiments, polymers can be substantially cross-linked to one another. In some embodiments, polymers can be substantially free of crosslinks. In some embodiments, polymers can be used in accordance with the present invention without undergoing a cross-linking step. It is further to be understood that the synthetic nanocarriers may comprise block copolymers, graft copolymers, blends, mixtures, and/or adducts of any of the foregoing and other polymers. Those skilled in the art will recognize that the polymers listed herein represent an exemplary, not comprehensive, list of polymers that can be of use in accordance with the present invention provided they meet the desired criteria.

The properties of these and other polymers and methods for preparing them are well known in the art (see, for example, U.S. Patents 6,123,727; 5,804,178; 5,770,417; 5,736,372; 5,716,404; 6,095,148; 5,837,752; 5,902,599; 5,696,175; 5,514,378; 5,512,600; 5,399,665; 5,019,379; 5,010,167; 4,806,621; 4,638,045; and 4,946,929; Wang et al., 2001, J. Am. Chem. Soc., 123:9480; Lim et al., 2001, J. Am. Chem. Soc., 123:2460; Langer, 2000, Acc. Chem. Res., 33:94; Langer, 1999, J. Control. Release, 62:7; and Uhrich et al., 1999, Chem. Rev., 99:3181). More generally, a variety of methods for synthesizing certain suitable polymers are described in Concise Encyclopedia of Polymer Science and Polymeric Amines and Ammonium Salts, Ed. by Goethals, Pergamon Press, 1980; Principles of Polymerization by Odian, John Wiley & Sons, Fourth Edition, 2004; Contemporary Polymer Chemistry by Allcock et al., Prentice-Hall, 1981; Deming et al., 1997, Nature, 390:386; and in U.S. Patents 6,506,577, 6,632,922, 6,686,446, and 6,818,732.

A wide variety of synthetic nanocarriers can be used according to the invention. In some embodiments, synthetic nanocarriers are spheres or spheroids. In some embodiments, synthetic nanocarriers are flat or plate-shaped. In some embodiments, synthetic nanocarriers are cubes or cubic. In some embodiments, synthetic nanocarriers are ovals or ellipses. In some embodiments, synthetic nanocarriers are cylinders, cones, or pyramids.

In some embodiments, it is desirable to use a population of synthetic nanocarriers that is relatively uniform in terms of size or shape so that each synthetic nanocarrier has similar properties. For example, at least 80%, at least 90%, or at least 95% of the synthetic nanocarriers, based on the total number of synthetic nanocarriers, may have a minimum dimension or maximum dimension that falls within 5%, 10%, or 20% of the average diameter or average dimension of the synthetic nanocarriers.

Compositions according to the invention can comprise elements in combination with pharmaceutically acceptable excipients, such as preservatives, buffers, saline, or phosphate buffered saline. The compositions may be made using conventional pharmaceutical manufacturing and compounding techniques to arrive at useful dosage forms. In an embodiment, compositions, such as those comprising the synthetic nanocarriers are suspended in sterile saline solution for injection together with a preservative.

In some embodiments, any component of the synthetic nanocarriers as provided herein may be isolated. Isolated refers to the element being separated from its native environment and present in sufficient quantities to permit its identification or use. This means, for example, the element may be purified as by chromatography or electrophoresis. Isolated elements may be, but need not be, substantially pure. Because an isolated element may be admixed with a pharmaceutically acceptable excipient in a pharmaceutical preparation, the element may comprise only a small percentage by weight of the preparation. The element is nonetheless isolated in that it has been separated from the substances with which it may be associated in living systems, *i.e.,* isolated from other lipids or proteins. Any of the elements provided herein may be isolated and included in the compositions or used in the methods in isolated form.

### D. METHODS OF MAKING AND USING THE COMPOSITIONS AND RELATED METHODS

Synthetic nanocarriers may be prepared using a wide variety of methods known in the art. For example, synthetic nanocarriers can be formed by methods such as nanoprecipitation, flow focusing using fluidic channels, spray drying, single and double emulsion solvent evaporation, solvent extraction, phase separation, milling (including cryomilling), supercritical fluid (such as supercritical carbon dioxide) processing, microemulsion procedures, microfabrication, nanofabrication, sacrificial layers, simple and complex coacervation, and other methods well known to those of ordinary skill in the art. Alternatively or additionally, aqueous and organic solvent syntheses for monodisperse semiconductor, conductive, magnetic, organic, and other nanomaterials have been described (Pellegrino et al., 2005, Small, 1:48; Murray et al., 2000, Ann. Rev. Mat. Sci., 30:545; and Trindade et al., 2001, Chem. Mat., 13:3843). Additional methods have been described in the literature (see, e.g., Doubrow, Ed., "Microcapsules and Nanoparticles in Medicine and Pharmacy," CRC Press, Boca Raton, 1992; Mathiowitz et al., 1987, J. Control. Release, 5:13; Mathiowitz et al., 1987, Reactive Polymers, 6:275; and Mathiowitz et al., 1988, J. Appl. Polymer Sci., 35:755; US Patents 5578325 and 6007845; P. Paolicelli et al., "Surface-modified PLGA-based Nanoparticles that can Efficiently Associate and Deliver Virus-like Particles" Nanomedicine. 5(6):843-853 (2010)).

Various materials may be encapsulated into synthetic nanocarriers as desirable using a variety of methods including but not limited to C. Astete et al., "Synthesis and characterization of PLGA nanoparticles" J. Biomater. Sci. Polymer Edn, Vol. 17, No. 3, pp. 247-289 (2006); K. Avgoustakis "Pegylated Poly(Lactide) and Poly(Lactide-Co-Glycolide) Nanoparticles: Preparation, Properties and Possible Applications in Drug Delivery" Current Drug Delivery 1:321-333 (2004); C. Reis et al., "Nanoencapsulation I. Methods for preparation of drug-loaded polymeric nanoparticles" Nanomedicine 2:8- 21 (2006); P. Paolicelli et al., "Surface-modified PLGA-based Nanoparticles that can Efficiently Associate and Deliver Virus-like Particles" Nanomedicine. 5(6):843-853 (2010). Other methods suitable for encapsulating materials into synthetic nanocarriers may be used, including without limitation methods disclosed in United States Patent 6,632,671 to Unger issued October 14, 2003.

In certain embodiments, synthetic nanocarriers are prepared by a nanoprecipitation process or spray drying. Conditions used in preparing synthetic nanocarriers may be altered to yield particles of a desired size or property (e.g., hydrophobicity, hydrophilicity, external morphology, "stickiness," shape, etc.). The method of preparing the synthetic nanocarriers and the conditions (e.g., solvent, temperature, concentration, air flow rate, etc.) used may depend on the materials to be included in the synthetic nanocarriers and/or the composition of the carrier matrix.

If synthetic nanocarriers prepared by any of the above methods have a size range outside of the desired range, such synthetic nanocarriers can be sized, for example, using a sieve.

In embodiments, the synthetic nanocarriers can be combined with an antigen or other composition by admixing in the same vehicle or delivery system.

Compositions provided herein may comprise inorganic or organic buffers (*e.g*., sodium or potassium salts of phosphate, carbonate, acetate, or citrate) and pH adjustment agents (*e.g*., hydrochloric acid, sodium or potassium hydroxide, salts of citrate or acetate, amino acids and their salts) antioxidants (*e.g.,* ascorbic acid, alpha-tocopherol), surfactants (*e.g.,* polysorbate 20, polysorbate 80, polyoxyethylene9-10 nonyl phenol, sodium desoxycholate), solution and/or cryo/lyo stabilizers (*e.g.,* sucrose, lactose, mannitol, trehalose), osmotic adjustment agents (*e.g.,* salts or sugars), antibacterial agents (*e.g*., benzoic acid, phenol, gentamicin), antifoaming agents (*e.g.,* polydimethylsilozone), preservatives (*e.g.,* thimerosal, 2-phenoxyethanol, EDTA), polymeric stabilizers and viscosity-adjustment agents (*e.g*., polyvinylpyrrolidone, poloxamer 488, carboxymethylcellulose) and co-solvents (*e.g*., glycerol, polyethylene glycol, ethanol).

Compositions according to the invention may comprise pharmaceutically acceptable excipients. The compositions may be made using conventional pharmaceutical manufacturing and compounding techniques to arrive at useful dosage forms. Techniques suitable for use in practicing the present invention may be found in Handbook of Industrial Mixing: Science and Practice, Edited by Edward L. Paul, Victor A. Atiemo-Obeng, and Suzanne M. Kresta, 2004 John Wiley & Sons, Inc.; and Pharmaceutics: The Science of Dosage Form Design, 2nd Ed. Edited by M. E. Auten, 2001, Churchill Livingstone. In an embodiment, compositions are suspended in a sterile saline solution for injection together with a preservative.

It is to be understood that the compositions of the invention can be made in any suitable manner, and the invention is in no way limited to compositions that can be produced using the methods described herein. Selection of an appropriate method of manufacture may require attention to the properties of the particular elements being associated.

In some embodiments, compositions are manufactured under sterile conditions or are initially or terminally sterilized. This can ensure that resulting compositions are sterile and noninfectious, thus improving safety when compared to non-sterile compositions. This provides a valuable safety measure, especially when subjects receiving the compositions have immune defects, are suffering from infection, and/or are susceptible to infection. In some embodiments, the compositions may be lyophilized and stored in suspension or as lyophilized powder depending on the formulation strategy for extended periods without losing activity.

Administration according to the present invention may be by a variety of routes, including but not limited to intradermal, intramuscular, subcutaneous, intravenous, and intraperitoneal routes. The compositions referred to herein may be manufactured and prepared for administration using conventional methods.

The compositions of the invention can be administered in effective amounts, such as the effective amounts described elsewhere herein. Doses of dosage forms may contain varying amounts of elements according to the invention. The amount of elements present in the inventive dosage forms can be varied according to their nature, the therapeutic benefit to be accomplished, and other such parameters. In embodiments, dose ranging studies can be conducted to establish optimal therapeutic amounts to be present in the dosage form. In embodiments, the elements are present in the dosage form in an amount effective to generate a desired effect and/or a reduced immune response upon administration to a subject. It may be possible to determine amounts to achieve a desired result using conventional dose ranging studies and techniques in subjects. Inventive dosage forms may be administered at a variety of frequencies. In an embodiment, at least one administration of the compositions provided herein is sufficient to generate a pharmacologically relevant response.

Another aspect of the disclosure relates to kits. In some embodiments, the kit comprises any one of the compositions provided herein. In some embodiments of any one of the kits provided, the kit comprises a rapalog, a hydrophobic carrier material and non-ionic surfactant with a hydrophilic-lipophilic balance (HLB) value less than or equal to 10. In some embodiments of any one of the kits provided, the amounts of the rapalog, hydrophobic carrier material and non-ionic surfactant with a hydrophilic-lipophilic balance (HLB) value less than or equal to 10 are in any one of the amounts provided herein for that component, respectively. In some embodiments of any one of the kits provided, the kit further comprises an antigen. In some embodiments of any one of the kits provided, the compositions or elements thereof can be contained within separate containers or within the same container in the kit. In some embodiments of any one of the kits provided, the container is a vial or an ampoule. In some embodiments of any one of the kits provided, the compositions or elements thereof are contained within a solution separate from the container, such that the compositions or elements may be added to the container at a subsequent time. In some embodiments of any one of the kits provided, the compositions or elements thereof are in lyophilized form each in a separate container or in the same container, such that they may be reconstituted at a subsequent time. In some embodiments of any one of the kits provided, the kit further comprises instructions for reconstitution, mixing, administration, etc. In some embodiments of any one of the kits provided, the instructions include a description of the methods described herein. Instructions can be in any suitable form, *e.g.,* as a printed insert or a label. In some embodiments of any one of the kits provided herein, the kit further comprises one or more syringes or other device(s) that can deliver synthetic nanocarriers *in vivo* to a subject.

The technology described herein may be futher defined by any of the following clauses:
1. A composition comprising synthetic nanocarriers comprising:
   a hydrophobic carrier material,
   a rapalog, and
   a non-ionic surfactant with a hydrophilic-lipophilic balance (HLB) value less than or equal to 10;
   wherein the amount of non-ionic surfactant with HLB value less than or equal to 10 is ≥ 0.01 but ≤ 20 weight% non-ionic surfactant with a HLB value less than or equal to 10/weight hydrophobic carrier material.
2. The composition of clause 1, wherein the composition is initially sterile filterable through a 0.22 µm filter.
3. The composition of clause 1 or 2, wherein the non-ionic surfactant with HLB value less than or equal to 10 is a non-ionic surfactant with HLB value less than 10.
4. The composition of clause 3, wherein the non-ionic surfactant with HLB value less than or equal to 10 is a non-ionic surfactant with HLB value less than 9.
5. The composition of clause 4, wherein the non-ionic surfactant with HLB value less than or equal to 10 is a non-ionic surfactant with HLB value less than 8.
6. The composition of clause 5, wherein the non-ionic surfactant with HLB value less than or equal to 10 is a non-ionic surfactant with HLB value less than 7.
7. The composition of clause 6, wherein the non-ionic surfactant with HLB value less than or equal to 10 is a non-ionic surfactant with HLB value less than 6.
8. The composition of clause 7, wherein the non-ionic surfactant with HLB value less than or equal to 10 is a non-ionic surfactant with HLB value less than 5.
9. The composition of any one of the preceding clauses, wherein the non-ionic surfactant with HLB value less than or equal to 10 comprises a sorbitan ester, fatty alcohol, fatty acid ester, ethoxylated fatty alcohol, poloxamer or a fatty acid.
10. The composition of clause 9, wherein the non-ionic surfactant with HLB value less than or equal to 10 comprises SPAN 40, SPAN 20, oleyl alcohol, stearyl alcohol, isopropyl palmitate, glycerol monostearate, BRIJ 52, BRIJ 93, Pluronic P-123, Pluronic L-31, palmitic acid, dodecanoic acid, glyceryl tripalmitate or glyceryl trilinoleate.
11. The composition of clause 10, wherein the non-ionic surfactant with HLB value less than or equal to 10 is SPAN 40.
12. The composition of any one of the preceding clauses, wherein the non-ionic surfactant with HLB value less than or equal to 10 is encapsulated in the synthetic nanocarriers, present on the surface of the synthetic nanocarriers, or both.
13. The composition of clause 12, wherein the amount of non-ionic surfactant with HLB value less than or equal to 10 is ≥ 0.1 but ≤ 15 weight% non-ionic surfactant with a HLB value less than or equal to 10/weight hydrophobic carrier material.
14. The composition of clause 13, wherein the amount of non-ionic surfactant with HLB value less than or equal to 10 is ≥ 1 but ≤ 13 weight% non-ionic surfactant with a HLB value less than or equal to 10/weight hydrophobic carrier material.
15. The composition of clause 13, wherein the amount of non-ionic surfactant with HLB value less than or equal to 10 is ≥ 1 but ≤ 9 weight% non-ionic surfactant with a HLB value less than or equal to 10/weight hydrophobic carrier material.
16. The composition of any one of the preceding clauses, wherein the hydrophobic carrier material comprises one or more hydrophobic polymers or lipids.
17. The composition of clause 16, wherein the hydrophobic carrier material comprises one or more hydrophobic polymers, and wherein the one or more hydrophobic polymers comprise a polyester.
18. The composition of clause 17, wherein the polyester comprises PLA, PLG, PLGA or polycaprolactone.
19. The composition of clause 17 or 18, wherein the hydrophobic carrier material comprises or further comprises PLA-PEG, PLGA-PEG or PCL-PEG.
20. The composition of any one of the preceding clauses, wherein the amount of hydrophobic carrier material in the synthetic nanocarriers is 5-95 weight% hydrophobic carrier material/total solids.
21. The composition of clause 20, wherein the amount of hydrophobic carrier material in the synthetic nanocarriers is 60-95 weight% hydrophobic carrier material/total solids.
22. The composition of any one of the preceding clauses, wherein the amount of rapalog is ≥ 6 but ≤ 50 weight% rapalog/weight hydrophobic carrier material.
23. The composition of clause 22, wherein the amount of rapalog is ≥ 7 but ≤ 30 weight% rapalog/weight hydrophobic carrier material.
24. The composition of clause 23, wherein the amount of rapalog is ≥ 8 but ≤ 24 weight% rapalog/weight hydrophobic carrier material.
25. The composition of any one of the preceding clauses, wherein the rapalog is encapsulated in the synthetic nanocarriers.
26. The composition of any one of the preceding clauses, wherein the rapalog is rapamycin.
27. The composition of any one of the preceding clauses, wherein the composition further comprises an antigen.
28. The composition of clause 27, wherein the antigen is admixed with the synthetic nanocarriers in the composition.
29. The composition of any one of the preceding clauses, wherein the mean of a particle size distribution obtained using dynamic light scattering of the synthetic nanocarriers is a diameter greater than 120nm.
30. The composition of clause 29, wherein the diameter is greater than 150nm.
31. The composition of clause 30, wherein the diameter is greater than 200nm.
32. The composition of clause 31, wherein the diameter is greater than 250nm.
33. The composition of any one of clauses 29-32, wherein the diameter is less than 300nm.
34. The composition of any one of clauses 29-31, wherein the diameter is less than 250nm.
35. The composition of clause 29 or 30, wherein the diameter is less than 200nm.
36. The composition of any one of the preceding clauses, wherein the composition further comprises a pharmaceutically acceptable carrier.
37. A kit comprising:
   the composition of any one of the preceding clauses for use in any one of the methods provided herein.
38. The kit of clause 37, wherein, when the composition does not comprise antigen, the kit further comprises an antigen.
39. The kit of clause 38, wherein the composition and antigen are contained in separate containers.
40. The kit of clause 38, wherein the composition and antigen are contained in the same container.
41. The kit of any one of clauses 37-40, further comprising instructions for use.
42. The kit of clause 41, wherein the instructions for use include a description of any one of the methods provided herein.
43. A method comprising administering the composition of any one of clauses 1-36 to a subject.
44. The method of clause 43, wherein, when the composition does not comprise antigen, the method further comprises administering antigen to the subject.
45. The method of clause 44, wherein the antigen is comprised in different synthetic nanocarriers.
46. The method of clause 45, wherein the antigen is not coupled to any synthetic nanocarriers.
47. The method of any one of clauses 43-46, wherein the administering is by intradermal, intramuscular, intravenous, intraperitoneal or subcutaneous administration.
48. A method for producing synthetic nanocarriers comprising a non-ionic surfactant with HLB value less than or equal to 10 and a rapalog, comprising:
   obtaining or providing a hydrophobic carrier material,
   obtaining or providing a non-ionic surfactant with HLB value less than or equal to 10,
   obtaining or providing a rapalog, and
   combining the hydrophobic carrier material, the non-ionic surfactant with HLB value less than or equal to 10 and the rapalog to form synthetic nanocarriers,
   wherein the amount of non-ionic surfactant with HLB value less than or equal to 10 in the synthetic nanocarriers is ≥ 0.01 but ≤ 20 weight% non-ionic surfactant with a HLB value less than or equal to 10/weight hydrophobic carrier material.
49. The method of clause 48, further comprising:
   dissolving the hydrophobic carrier material, non-ionic surfactant with HLB value less than or equal to 10 and rapalog in a solvent;
   obtaining or providing another surfactant;
   forming a first and then second O/W emulsion with the dissolved hydrophobic carrier material, non-ionic surfactant with HLB value less than or equal to 10 and rapalog, and other surfactant;
   mixing the first and second O/W emulsions; and
   allowing the solvent to evaporate.
50. The method of clause 49, wherein the solvent is dichloromethane, ethyl acetate, chloroform, or propylene carbonate.
51. The method of any one of clauses 48-50, wherein the non-ionic surfactant with HLB value less than or equal to 10 is a non-ionic surfactant with HLB value less than 10.
52. The method of clause 51, wherein the non-ionic surfactant with HLB value less than or equal to 10 is a non-ionic surfactant with HLB value less than 9.
53. The method of clause 52, wherein the non-ionic surfactant with HLB value less than or equal to 10 is a non-ionic surfactant with HLB value less than 8.
54. The method of clause 53, wherein the non-ionic surfactant with HLB value less than or equal to 10 is a non-ionic surfactant with HLB value less than 7.
55. The method of clause 54, wherein the non-ionic surfactant with HLB value less than or equal to 10 is a non-ionic surfactant with HLB value less than 6.
56. The method of clause 55, wherein the non-ionic surfactant with HLB value less than or equal to 10 is a non-ionic surfactant with HLB value less than 5.
57. The method of any one of clauses 48-56, wherein the non-ionic surfactant with HLB value less than or equal to 10 comprises a sorbitan ester, fatty alcohol, fatty acid ester, ethoxylated fatty alcohol, poloxamer or a fatty acid.
58. The method of clause 57, wherein the non-ionic surfactant with HLB value less than or equal to 10 comprises SPAN 40, SPAN 20, oleyl alcohol, stearyl alcohol, isopropyl palmitate, glycerol monostearate, BRIJ 52, BRIJ 93, Pluronic P-123, Pluronic L-31, palmitic acid, dodecanoic acid, glyceryl tripalmitate or glyceryl trilinoleate.
59. The method of clause 58, wherein the non-ionic surfactant with HLB value less than or equal to 10 is SPAN 40.
60. The method of clause 59, wherein the amount of non-ionic surfactant with HLB value less than or equal to 10 is ≥ 0.1 but ≤ 15 weight% non-ionic surfactant with a HLB value less than or equal to 10/weight hydrophobic carrier material.
61. The method of clause 60, wherein the amount of non-ionic surfactant with HLB value less than or equal to 10 is ≥ 1 but ≤ 13 weight% non-ionic surfactant with a HLB value less than or equal to 10/weight hydrophobic carrier material.
62. The method of clause 61, wherein the amount of non-ionic surfactant with HLB value less than or equal to 10 is ≥ 1 but ≤ 9 weight% non-ionic surfactant with a HLB value less than or equal to 10/weight hydrophobic carrier material.
63. The method of any one of clauses 48-62, wherein the hydrophobic carrier material comprises one or more hydrophobic polymers or lipids.
64. The method of clause 63, wherein the hydrophobic carrier material comprises one or more hydrophobic polymers, and wherein the one or more hydrophobic polymers comprise a polyester.
65. The method of clause 64, wherein the polyester comprises PLA, PLG, PLGA or polycaprolactone.
66. The method of clause 64 or 65, wherein the hydrophobic carrier material comprises or further comprises PLA-PEG, PLGA-PEG or PCL-PEG.
67. The composition of any one of clauses 48-66, wherein the amount of hydrophobic carrier material in the synthetic nanocarriers is 5-95 weight% hydrophobic carrier material/total solids.
68. The method of clause 67, wherein the amount of hydrophobic carrier material in the synthetic nanocarriers is 60-95 weight% hydrophobic carrier material/total solids.
69. The method of any one of clauses 48-68, wherein the amount of rapalog is ≥ 6 but ≤ 50 weight% rapalog/weight hydrophobic carrier material.
70. The method of clause 69, wherein the amount of rapalog is ≥ 7 but ≤ 30 weight% rapalog/weight hydrophobic carrier material.
71. The method of clause 70, wherein the amount of rapalog is ≥ 8 but ≤ 24 weight% rapalog/weight hydrophobic carrier material.
71. The method of any one of clauses 48-70, wherein the rapalog is rapamycin.
72. The method of any one of clauses 48-71, wherein the method further comprises filtering the resulting composition.
73. The method of clause 72, wherein the filtering comprises filtering through a 0.22 µm filter.
74. The composition produced by the method of any one of clauses 48-73.
75. A method comprising administering the composition of clause 74.

### EXAMPLES

### Example 1 - Low HLB Surfactant, SM, Increases RAPA Loading and Synthetic Nanocarrier Filterability

Nanocarrier compositions containing the polymers PLA (inherent viscosity 0.41 dL/g) and PLA-PEG (5 kDa PEG block, inherent viscosity 0.50 dL/g) as well as the hydrophobic drug rapamycin (RAPA) were synthesized, with or without the addition of the low HLB surfactant sorbitan monopalmitate (SM), using the oil-in-water emulsion evaporation method. The organic phase was formed by dissolving the polymers and RAPA in dichloromethane. The emulsion was formed by homogenizing the organic phase in an aqueous phase containing the surfactant PVA using a probe-tip sonicator. The emulsion was then combined with a larger amount of aqueous buffer and mixed to allow dissolution and evaporation of the solvent. The resulting nanocarriers were washed and filtered through a 0.22 µm filter. All compositions contained 100 mg of polymer. The RAPA content in the different compositions was varied.

| Sample ID | RAPA Added to Composition (mg) | SM Added to Composition (mg) | Unwashed Diameter (nm) | Final Diameter (nm) | RAPA Load (%) | Filter Throughput (g/m²) |
|---|---|---|---|---|---|---|
| 1 | 12.2 | 0 | 148 | 148 | 6.1 | 80 |
| 2 | 13.3 | 0 | 171 | 151 | 6.2 | 28 |
| 3 | 14.3 | 0 | 202 | 154 | 5.8 | 16 |
| 4 | 13.6 | 5 | 156 | 161 | 9.2 | >174 |
| 5 | 17 | 5 | 168 | 170 | 11.8 | >184 |
| 6 | 20.4 | 5 | 181 | 179 | 14.9 | 77 |

For the compositions not containing the surfactant SM (samples 1, 2, and 3), several indications of a limiting ability to fully incorporate RAPA in the nanocarrier composition were observed as increasing amounts of RAPA were added. The increasing difference between the pre- and post-filtration nanocarrier sizes at the higher RAPA formulation levels in the absence of SM were indicative of the presence of larger particulates (individual particles or aggregates) being removed during the washing and/or filtration processes. This was also indicated by the decreased filter throughput before clogging. Finally, adding increasing amounts of RAPA to nanocarrier compositions without SM did not result in increased RAPA loading (for example, sample 1 compared to sample 3), indicating that the additional RAPA was separable from the bulk of the nanocarriers and was removed during the washing and/or filtration steps.

By contrast, the compositions containing the surfactant SM readily incorporated increased amounts of RAPA. The nanocarrier size was not affected by filtration, and increasing the amount of RAPA added to the composition resulted in increased RAPA loading of the nanocarriers. Some filter throughput reduction was observed at the highest loading level (sample 6), but this may be due to the inherently larger nanocarrier size. In sum, the incorporation of SM helped to increase RAPA loading and filterability of the synthetic nanocarrier compositions.

### Example 2 - SM and Cholesterol Increased RAPA Loading and Filterability

Nanocarrier compositions were produced using the materials and methods as described in **Example 1.** Nanocarriers containing polymer and RAPA were produced with varying RAPA load levels. In addition, nanocarriers highly loaded with RAPA were also produced using an excipient, the surfactant SM or cholesterol, in an excipient:RAPA mass ratio of 3.2:1.

| Sample ID | Excipient | Diameter (nm) | RAPA Load (%) | Filter Throughput (g/m²) |
|---|---|---|---|---|
| 7 | - | 131 | 5.6 | >148 |
| 8 | - | 138 | 7.9 | 37 |
| 9 | SM | 165 | 9.3 | >178 |
| 10 | cholesterol | 166 | 14.3 | >180 |

The samples of nanocarriers produced in the absence of excipients (samples 7 and 8) demonstrated that the increase in RAPA loading beyond a point of apparent nanocarrier saturation tends to lead to a reduction in filter throughput. The addition of either SM or cholesterol resulted in greater RAPA loading while maintaining stability (samples 9 and 10).

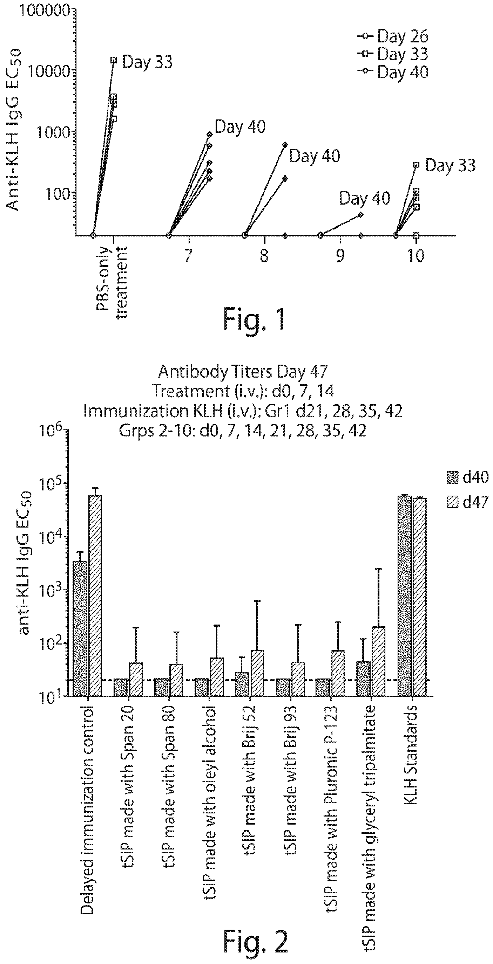

To assess the ability of the compositions to induce immune tolerance, mice were intravenously injected three times weekly with co-administered nanocarrier and KLH (keyhole limpet hemocyanin) with the same RAPA dose, and then challenged weekly with KLH only. The sera of the mice were then analyzed for antibodies to KLH after each KLH challenge (**Fig. 1**).

While all mice receiving RAPA nanocarrier treatment received the same doses of RAPA, the different groups show different degrees of tolerization to KLH. All 5 mice that received the nanocarrier compositions with the lowest load (sample 7) had quantifiable titers of anti-KHL antibodies after the third KLH challenge (at day 40). This group of mice developed reduced titers of anti-KLH antibodies compared to the mice that received PBS only, but exhibited the least tolerization compared to the other nanocarrier groups. Increasing the RAPA load of the nanocarriers in the absence of an excipient (SM or cholesterol) (sample 8) significantly improved tolerization, with only 2 of 5 mice demonstrating quantifiable titers after 3 KLH challenges (at day 40). The composition containing cholesterol as the excipient (sample 10), despite the high RAPA loading of the nanocarriers, resulted in four out of five mice demonstrating significant anti-KLH antibody titers after only two challenges (at day 33). The nanocarrier composition containing SM (sample 9) demonstrated both high-throughput 0.22 µm filter throughput during production and superior tolerization, with only one out of five mice developing a quantifiable anti-KLH antibody titer after three KLH challenges (at day 40). The results of this study indicate that both excipients (SM and cholesterol) enabled increased nanocarrier loading consistent with tolerance-inducing performance and processing favorability as indicated by filtration throughput. The low HLB surfactant SM provided the properties needed to increase stability of the nanocarriers and demonstrated higher performance.

### Example 3 - Effects of Low HLB Surfactant on RAPA Load and Filterability

### Materials and Methods

PLA with an inherent viscosity of 0.41 dL/g was purchased from Lakeshore Biomaterials (756 Tom Martin Drive, Birmingham, AL 35211), product code 100 DL 4A. PLA-PEG-OMe block co-polymer with a methyl ether terminated PEG block of approximately 5,000 Da and an overall inherent viscosity of 0.50 DL/g was purchased from Lakeshore Biomaterials (756 Tom Martin Drive, Birmingham, AL 35211), product code 100 DL mPEG 5000 5CE. Rapamycin was purchased from Concord Biotech Limited (1482-1486 Trasad Road, Dholka 382225, Ahmedabad India), product code SIROLIMUS. EMPROVE^{®} Polyvinyl Alcohol 4-88, USP (85-89% hydrolyzed, viscosity of 3.4-4.6 mPa s) was purchased from EMD Chemicals Inc. (480 South Democrat Road Gibbstown, NJ 08027), product code 1.41350. Dulbecco's phosphate buffered saline 1X (DPBS) was purchased from Lonza (Muenchensteinerstrasse 38, CH-4002 Basel, Switzerland), product code 17-512Q. Sorbitan monopalmitate was purchased from Croda International (300-A Columbus Circle, Edison, NJ 08837), product code SPAN 40. Polysorbate 80 was purchased from NOF America Corporation (One North Broadway, Suite 912 White Plains, NY 10601), product code Polysorbate80 (HX2). Sorbitan monolaurate (SPAN 20) was purchased from Alfa Aesar (26 Parkridge Rd Ward Hill, MA 01835), product code L12099. Sorbitan stearate (SPAN 60) was purchased from Sigma-Aldrich (3050 Spruce St. St. Louis, MO 63103), product code S7010. Sorbitan monooleate (SPAN 80) was purchased from Tokyo Chemical Industry Co., Ltd. (9211 North Harborgate Street Portland, OR 97203), product code S0060. Octyl β-D-glucopyranoside was purchased from Sigma-Aldrich (3050 Spruce St. St. Louis, MO 63103), product code 08001. Oleyl alcohol was purchased from Alfa Aesar (26 Parkridge Rd Ward Hill, MA 01835), product code A18018. Isopropyl palmitate was purchased from Sigma-Aldrich (3050 Spruce St. St. Louis, MO 63103), product code W515604. Polyethylene glycol hexadecyl ether (BRIJ 52) was purchased from Sigma-Aldrich (3050 Spruce St. St. Louis, MO 63103), product code 388831. Polyethylene glycol oleyl ether (BRIJ 93) was purchased from Sigma-Aldrich (3050 Spruce St. St. Louis, MO 63103), product code 388866. Poly(ethylene glycol)-*block*-poly(propylene glycol)*-block*-poly(ethylene glycol) (Pluronic L-31) was purchased from Sigma-Aldrich (3050 Spruce St. St. Louis, MO 63103), product code 435406. Poly(ethylene glycol)-*block*-poly(propylene glycol)-*block-*poly(ethylene glycol) (Pluronic P-123) was purchased from Sigma-Aldrich (3050 Spruce St. St. Louis, MO 63103), product code 435465. Palmitic Acid was purchased from Sigma-Aldrich (3050 Spruce St. St. Louis, MO 63103), product code P0500. DL-α-palmitin was purchased from Sigma-Aldrich (3050 Spruce St. St. Louis, MO 63103), product code M1640. Glyceryl Tripalmitate was purchased from Sigma-Aldrich (3050 Spruce St. St. Louis, MO 63103), product code T5888.

For Sample 11, solutions were prepared as follows:
**Solution 1:** A polymer and rapamycin mixture was prepared by dissolving PLA at 75 mg/mL, PLA-PEG-Ome at 25 mg/mL, and rapamycin at 16 mg/mL in dichloromethane. **Solution 2**: A Polysorbate80 mixture was prepared by dissolving Polysorbate80 at 80 mg/mL in dichloromethane. **Solution 3:** Polyvinyl alcohol was prepared at 50 mg/mL in 100 mM pH 8 phosphate buffer.

An O/W emulsion was prepared by combining Solution 1 (0.5 mL), Solution 2 (0.1 mL), dichloromethane (0.4 mL) and Solution 3 (3.0 mL) in a small glass pressure tube, vortex mixed for 10 seconds, and was then sonicated at 30% amplitude for 1 minute with the pressure tube immersed in an ice water bath, using a Branson Digital Sonifier 250. The emulsion was then added to a 50 mL beaker containing DPBS (30 mL). A second O/W emulsion was prepared using the same materials and method as above and then added to the same container containing the first emulsion and DPBS. This was then stirred at room temperature for 2 hours to allow the dichloromethane to evaporate and for the nanocarriers to form. A portion of the nanocarriers was washed by transferring the nanocarrier suspension to a centrifuge tube and centrifuging at 75,600×g and 4 °C for 50 minutes, removing the supernatant, and re-suspended the pellet in DPBS containing 0.25% w/v PVA. The wash procedure was repeated and then the pellet was re-suspended in DPBS containing 0.25% w/v PVA to achieve a nanocarrier suspension having a nominal concentration of 10 mg/mL on a polymer basis. The nanocarrier suspension was then filtered using a 0.22 µm PES membrane syringe filter (Millipore part number SLGP033RB). The filtered nanocarrier suspension was then stored at -20°C.

For samples 12-25, solutions were prepared as follows:
**Solution 1:** A polymer and rapamycin mixture was prepared by dissolving PLA at 75 mg/mL, PLA-PEG-Ome at 25 mg/mL, and rapamycin at 16 mg/mL in dichloromethane. **Solution 2**: The HLB mixture was prepared by dissolving the HLB surfactant at 5.0 mg/mL in dichloromethane. HLB surfactants include SPAN 20, SPAN 40, SPAN 60, SPAN 80, octyl β-D-glucopyranoside, oleyl acid, isopropyl palmitate, BRIJ 52, BRIJ 93, Pluronic L-31, Pluronic P-123, palmitic acid, DL-α-palmitin, and glyceryl tripalmitate. **Solution 3:** Polyvinyl alcohol was prepared at 62.5 mg/mL in 100 mM pH 8 phosphate buffer.

An O/W emulsion was prepared by combining Solution 1 (0.5 mL), Solution 2 (0.5 mL), and Solution 3 (3.0 mL) in a small glass pressure tube, vortex mixed for 10 seconds, and was then sonicated at 30% amplitude for 1 minute with the pressure tube immersed in an ice water bath using a Branson Digital Sonifier 250. The emulsion was then added to a 50 mL beaker containing DPBS (30 mL). A second O/W emulsion was prepared using the same materials and method as above and then added to the same beaker containing the first emulsion and DPBS. This was then stirred at room temperature for 2 hours to allow the dichloromethane to evaporate and for the nanocarriers to form. A portion of the nanocarriers was washed by transferring the nanocarrier suspension to a centrifuge tube and centrifuging at 75,600×g and 4 °C for 50 minutes, removing the supernatant, and re-suspended the pellet in DPBS containing 0.25% w/v PVA. The wash procedure was repeated and then the pellet was re-suspended in DPBS containing 0.25% w/v PVA to achieve a nanocarrier suspension having a nominal concentration of 10 mg/mL on a polymer basis. The nanocarrier suspension was then filtered using a 0.22 µm PES membrane syringe filter (Millipore part number SLGP033RB). The filtered nanocarrier suspension was then stored at - 20°C.

| Sample | Organic Phase Surfactant | HLB of Surfactant | Size (nm) | Filtration | Number Filters | Calculated g NP/m² | Yield (%) | Rapamycin Load (%) |
|---|---|---|---|---|---|---|---|---|
| 11 | Polysorbate 80 | 15 | 184 | Millex 0.22 µm | >1 | 22 | 91 | 9.7 |
| 12 | SPAN 20 | 8.6 | 148 | Millex 0.22 µm | 1 | >144 | 71 | 11.2 |
| 13 | SPAN 40 | 6.7 | 149 | Millex 0.22 µm | 1 | >154 | 77 | 11.2 |
| 14 | SPAN 60 | 4.7 | 151 | Millex 0.22 µm | 1 | >154 | 77 | 11.0 |
| 15 | SPAN 80 | 4.3 | 144 | Millex 0.22 µm | 1 | >169 | 85 | 11.1 |
| 16 | octyl β-D-glucopyranoside | 12 | 127 | Millex 0.22 µm | 3 | 47 | 64 | 6.7 |
| 17 | oleyl alcohol | 1.3 | 165 | Millex 0.22 µm | 1 | >157 | 78 | 12.5 |
| 18 | isopropyl palmitate | 2.9 | 171 | Millex 0.22 µm | 1 | >144 | 71 | 10.9 |
| 19 | Brij 52 | 5 | 182 | Millex 0.22 µm | 1 | >138 | 77 | 11.2 |
| 20 | Brij 93 | 4 | 174 | Millex 0.22 µm | 1 | >158 | 79 | 11.9 |
| 21 | Pluronic L-31 | 1-7 | 169 | Millex 0.22 µm | 4 | 31 | 70 | 8.5 |
| 22 | Pluronic P-123 | 7-9 | 162 | Millex 0.22 µm | 1 | >145 | 72 | 10.7 |
| 23 | Palmitic Acid | 3.2 | 132 | Millex 0.22 µm | 1 | >141 | 71 | 1.0 |
| 24 | DL-α-palmitin | 7.2 | 153 | Millex 0.22 µm | 3 | 51 | 68 | 7.4 |
| 25 | Glyceryl Tripalmitate | 4.3 | 168 | Millex 0.22 µm | 1 | >146 | 73 | 10.0 |

The HLB for most of the low HLB surfactants was determined using publicly available information. For DL-α-Palmitin, the HLB was calculated using the following formula: Mw = 330.5 g/mol, hydrophilic portion = 119.0 g/mol; HLB = 119.0 / 330.5 * 100 / 5 = 7.2. For Glyceryl Palmitate, the HLB was calculated using the following formula: Mw = 807.3 g/mol, hydrophilic portion = 173.0 g/mol; HLB = 173.0 / 807.3 * 100 / 5 = 4.3. For Isopropyl Palmitate, the HLB was calculated using the following formula: Mw = 298.5 g/mol, hydrophilic portion = 44.0 g/mol; HLB = 44.0 / 298.5 * 100 / 5 = 2.9. For Oleyl Alcohol, the HLB was calculated using the following formula: Mw = 268.5 g/mol, hydrophilic portion =17.0 g/mol; HLB = 17.0 / 268.5 * 100 / 5 = 1.3. In addition, the load of low HLB surfactant was measured by extraction followed by quantitation by an HPLC method.

Prior to injection into animals, the bulk nanocarrier suspension was thawed in a room temperature water bath for 30 minutes. The nanocarriers were diluted with DPBS to reach a desired concentration of 278 µg/mL rapamycin. C57BL/6 female mice aged 6 weeks were treated intravenously on d0, 7, and 14 with nanocarriers (1.17 mL) mixed with 130 µL of 10x KLH (Keyhole limpet hemocyanin). The mice were boosted with 200 µg of KLH on days 21, 28, 35, and 42. Anti-KLH IgG titers (measured by ELISA) were read on days 40, 47, and 61. The results demonstrate that low HLB surfactant can result in substantial rapamycin loads and synthetic nanocarrier filterability. In addition, all of the nanocarriers with low HLB surfactants as shown in **Fig. 2** resulted in reduced antibody titers for at least 40 and 47 days.

### Example 4 - Effect of Low HLB Surfactant on Synthetic Nanocarrier Filterability

### Materials and Methods

PLA-PEG-OMe block co-polymer with a methyl ether terminated PEG block of approximately 5,000 Da and an overall inherent viscosity of 0.50 DL/g was purchased from Evonik Industries (Rellinghauser Straße 1-11 45128 Essen, Germany), product code 100 DL mPEG 5000 5CE. PLA with an inherent viscosity of 0.41 dL/g was purchased from Evonik Industries (Rellinghauser Straße 1-11 45128 Essen Germany), product code 100 DL 4A. Rapamycin was purchased from Concord Biotech Limited, 1482-1486 Trasad Road, Dholka 382225, Ahmedabad India. Product code SIROLIMUS. Everolimus was purchased from LC Laboratories (165 New Boston Street Woburn, MA 01801). Part Number E-4040. Temsirolimus was purchased from LC Laboratories (165 New Boston Street Woburn, MA 01801). Part number T-8040. Sorbitan monopalmitate was purchased from Croda (315 Cherry Lane New Castle Delaware 19720), product code SPAN 40. Dichloromethane was purchased from Spectrum (14422 S San Pedro Gardena CA, 90248-2027). Part number M1266. EMPROVE^{®} Polyvinyl Alcohol 4-88, USP (85-89% hydrolyzed, viscosity of 3.4-4.6 mPa·s) was purchased from EMD Chemicals Inc. (480 South Democrat Road Gibbstown, NJ 08027), product code 1.41350. Dulbecco's Phosphate Buffered Saline, 1X, 0.0095 M (PO4), without calcium and magnesium, was purchased from BioWhittaker (8316 West Route 24 Mapleton, IL 61547), part number #12001, product code Lonza DPBS. Emulsification was carried out using a Branson Digital Sonifier 250 with a 1/8" tapered tip titanium probe.

Solutions were prepared as follows:
**Solution 1:** A polymer mixture was prepared by dissolving PLA-PEG-OMe (100 DL mPEG 5000 5CE) at 50 mg per 1 mL and PLA (100 DL 4A) at 150 mg per mL in dichloromethane. **Solution 2:** Rapamycin was dissolved at 160 mg per 1 mL in dichloromethane. **Solution 3:** Everolimus was dissolved at 150 mg per mL in dichloromethane. **Solution 4:** Temsirolimus was dissolved at 150 mg per mL in dichloromethane. **Solution 5:** Sorbitan monopalmitate (SPAN 40) was dissolved at 50 mg per 1 mL in dichloromethane. **Solution 6:** Dichloromethane was sterile filtered using a 0.2 µm PTFE membrane syringe filter (VWR part number 28145-491). **Solution 7:** A polyvinyl alcohol solution was prepared by dissolving polyvinyl alcohol (EMPROVE^{®} Polyvinyl Alcohol 4-88) at 75 mg per 1 mL in 100 mM pH 8 phosphate buffer. **Solution 8:** A polyvinyl alcohol and Dulbecco's phosphate buffered saline, 1X, 0.0095 M (PO4) mixture was prepared by dissolving polyvinyl alcohol (EMPROVE^{®} Polyvinyl Alcohol 4-88) at 2.5 mg per 1 mL in Dulbecco's phosphate buffered saline, 1X, 0.0095 M (PO4) (Lonza DPBS).

For sample 26, an O/W emulsion was prepared by combining Solution 1 (0.5 mL), Solution 2 (0.1 mL), Solution 5 (0.1 mL), and Solution 6 (0.30 mL) in a small glass pressure tube. The solution was mixed by repeat pipetting. Next, Solution 7 (3.0 mL) was added, and the formulation was vortex mixed for ten seconds. The formulation was then sonicated with the pressure tube immersed in an ice bath for 1 minute at 30% amplitude. The emulsion was then added to an open 50 mL beaker containing Lonza DPBS (30 mL). This was then stirred at room temperature for 2 hours to allow the dichloromethane to evaporate and for the nanocarriers to form. A portion of the nanocarriers were washed by transferring the nanocarrier suspension to a centrifuge tube and centrifuging at 75,600×g and 4 °C for 50 minutes, removing the supernatant, and re-suspending the pellet in Solution 8. The wash procedure was repeated and then the pellet was re-suspended in Solution 8 to achieve a nanocarrier suspension having a nominal concentration of 10 mg per mL on a polymer basis. The nanocarrier formulation was filtered using a 0.22 µm PES membrane syringe filter (Millex part number SLGP033RS). The mass of the nanocarrier solution filter throughput was measured. The filtered nanocarrier solution was then stored at -20°C.

For sample 27, an O/W emulsion was prepared by combining Solution 1 (0.5 mL), Solution 2 (0.1 mL), and Solution 6 (0.40 mL) in a small glass pressure tube. The solution was mixed by repeat pipetting. Next, Solution 7 (3.0 mL) was added, and the formulation was vortex mixed for ten seconds. The formulation was then sonicated with the pressure tube immersed in an ice bath for 1 minute at 30% amplitude. The emulsion was then added to a 50 mL open beaker containing Lonza DPBS (30 mL). This was then stirred at room temperature for 2 hours to allow the dichloromethane to evaporate and for the nanocarriers to form. A portion of the nanocarriers were washed by transferring the nanocarrier suspension to a centrifuge tube and centrifuging at 75,600×g and 4 °C for 50 minutes, removing the supernatant, and re-suspending the pellet in Solution 8. The wash procedure was repeated and then the pellet was re-suspended in Solution 8 to achieve a nanocarrier suspension having a nominal concentration of 10 mg per mL on a polymer basis. The nanocarrier formulation was filtered using a 0.22 µm PES membrane syringe filter (Millex part number SLGP033RS). The mass of the nanocarrier solution filter throughput was measured. The filtered nanocarrier solution was then stored at -20°C.

For sample 28, an O/W emulsion was prepared by combining Solution 1 (0.5 mL), Solution 3 (0.2 mL), Solution 5 (0.1 mL), and Solution 6 (0.20 mL) in a small glass pressure tube. The solution was mixed by repeat pipetting. Next, Solution 7 (3.0 mL) was added, and the formulation was vortex mixed for ten seconds. The formulation was then sonicated with the pressure tube immersed in an ice bath for 1 minute at 30% amplitude. The emulsion was then added to an open 50 mL beaker containing Lonza DPBS (30 mL). This was then stirred at room temperature for 2 hours to allow the dichloromethane to evaporate and for the nanocarriers to form. A portion of the nanocarriers were washed by transferring the nanocarrier suspension to a centrifuge tube and centrifuging at 75,600×g and 4 °C for 50 minutes, removing the supernatant, and re-suspending the pellet in Solution 8. The wash procedure was repeated and then the pellet was re-suspended in Solution 8 to achieve a nanocarrier suspension having a nominal concentration of 10 mg per mL on a polymer basis. The nanocarrier formulation was filtered using a 0.22 µm PES membrane syringe filter (Millex part number SLGP033RS). The mass of the nanocarrier solution filter throughput was measured. The filtered nanocarrier solution was then stored at -20°C.

For sample 29, an O/W emulsion was prepared by combining Solution 1 (0.5 mL), Solution 3 (0.2 mL), and Solution 6 (0.30 mL) in a small glass pressure tube. The solution was mixed by repeat pipetting. Next, Solution 7 (3.0 mL) was added, and the formulation was vortex mixed for ten seconds. The formulation was then sonicated with the pressure tube immersed in an ice bath for 1 minute at 30% amplitude. The emulsion was then added to an open 50 mL beaker containing Lonza DPBS (30 mL). This was then stirred at room temperature for 2 hours to allow the dichloromethane to evaporate and for the nanocarriers to form. A portion of the nanocarriers were washed by transferring the nanocarrier suspension to a centrifuge tube and centrifuging at 75,600×g and 4 °C for 50 minutes, removing the supernatant, and re-suspending the pellet in Solution 8. The wash procedure was repeated and then the pellet was re-suspended in Solution 8 to achieve a nanocarrier suspension having a nominal concentration of 10 mg per mL on a polymer basis. The nanocarrier formulation was filtered using a 0.22µm PES membrane syringe filter (Millex part number SLGP033RS). The mass of the nanocarrier solution filter throughput was measured. The filtered nanocarrier solution was then stored at -20°C.

For sample 30, an O/W emulsion was prepared by combining Solution 1 (0.5 mL), Solution 4 (0.2 mL), Solution 5 (0.1 mL), and Solution 6 (0.20 mL) in a small glass pressure tube. The solution was mixed by repeat pipetting. Next, Solution 7 (3.0 mL) was added, and the formulation was vortex mixed for ten seconds. The formulation was then sonicated with the pressure tube immersed in an ice bath for 1 minute at 30% amplitude. The emulsion was then added to an open 50 mL beaker containing Lonza DPBS (30 mL). This was then stirred at room temperature for 2 hours to allow the dichloromethane to evaporate and for the nanocarriers to form. A portion of the nanocarriers were washed by transferring the nanocarrier suspension to a centrifuge tube and centrifuging at 75,600×g and 4 °C for 50 minutes, removing the supernatant, and re-suspending the pellet in Solution 8. The wash procedure was repeated and then the pellet was re-suspended in Solution 8 to achieve a nanocarrier suspension having a nominal concentration of 10 mg per mL on a polymer basis. The nanocarrier formulation was filtered using a 0.22 µm PES membrane syringe filter (Millex part number SLGP033RS). The mass of the nanocarrier solution filter throughput was measured. The filtered nanocarrier solution was then stored at -20°C.

For sample 31, an O/W emulsion was prepared by combining Solution 1 (0.5 mL), Solution 4 (0.2 mL), Solution 6 (0.30 mL) in a small glass pressure tube. The solution was mixed by repeat pipetting. Next, Solution 7 (3.0 mL) was added, and the formulation was vortex mixed for ten seconds. The formulation was then sonicated with the pressure tube immersed in an ice bath for 1 minute at 30% amplitude. The emulsion was then added to an open 50 mL beaker containing Lonza DPBS (30 mL). This was then stirred at room temperature for 2 hours to allow the dichloromethane to evaporate and for the nanocarriers to form. A portion of the nanocarriers were washed by transferring the nanocarrier suspension to a centrifuge tube and centrifuging at 75,600×g and 4 °C for 50 minutes, removing the supernatant, and re-suspending the pellet in Solution 8. The wash procedure was repeated and then the pellet was re-suspended in Solution 8 to achieve a nanocarrier suspension having a nominal concentration of 10 mg per mL on a polymer basis. The nanocarrier formulation was filtered using a 0.22 µm PES membrane syringe filter (Millex part number SLGP033RS). The mass of the nanocarrier solution filter throughput was measured. The filtered nanocarrier solution was then stored at -20°C.

Nanocarrier size was determined by dynamic light scattering. The amount of rapalog in the nanocarrier was determined by HPLC analysis. The total dry-nanocarrier mass per mL of suspension was determined by a gravimetric method. The filterability was evaluated by the amount of filtrate that passed through the first filter.

The data show that for a number of rapalogs, the incorporation of SPAN 40 in the synthetic nanocarriers resulted in an increase in filterability of the synthetic nanocarrier compositions.

| Nanocarrier ID | Rapalog | Low HLB Surfactant | Effective Diameter (nm) | Rapalog Content (% w/w) | Nanocarrier Yield (%) | 0.22µm Filter Throughput (g/m²) |
|---|---|---|---|---|---|---|
| 26 | Rapamycin | SPAN 40 | 179 | 17.19 | 80 | 98 |
| 27 | Rapamycin | None | 226 | 17.56 | 75 | 10 |
| 28 | Everolimus | SPAN 40 | 188 | 20.11 | 80 | 76 |
| 29 | Everolimus | None | 248 | 36.81 | 98 | 7 |
| 30 | Temsirolimus | SPAN 40 | 213 | 26.94 | 89 | 18 |
| 31 | Temsirolimus | None | 225 | 28.79 | 78 | 5 |

### Example 5 - Effects of SPAN 40 on Filterability of Synthetic Nanocarriers Comprising Non-Polyester Polymers

### Materials and Methods

Rapamycin was purchased from Concord Biotech Limited (1482-1486 Trasad Road, Dholka 382225, Ahmedabad India). Product code SIROLIMUS. Poly(styrene)-*block-*poly(ethylene glycol) (PS-PEG) with a methyl ether terminated PEG block of approximately 700-1,100 Da was purchased from Sigma Aldrich (3050 Spruce St. St. Louis, MO 63103). Part number 686476. Poly(styrene)-*block*-Poly(methyl methacrylate) (PS-PMMA) (Mw=30,000 Da) with a methacrylate: styrene ratio of 1:1 was purchased from Sigma Aldrich (3050 Spruce St. St. Louis, MO 63103). Part number 749184. 1,2-dimyristoyl-*sn*-glycero-3-phosphocholine (DMPC) was purchased from Avanti Polar Lipids, Inc (Avanti Polar Lipids, Inc. 700 Industrial Park Drive Alabaster, Alabama 35007-9105). Part number 850345P. Poly(ethylene glycol)-*block-*poly(propylene glycol)-*block-*poly(ethylene glycol) (P-123) with a PEG:PPG:PEG ratio of 20:70:20 was purchased from Sigma Aldrich (3050 Spruce St. St. Louis, MO 63103). Product code Pluronic^{®} P-123, part number 435465. Dichloromethane was purchased from Spectrum (14422 S San Pedro Gardena CA, 90248-2027). Part number M1266. Sorbitan monopalmitate was purchased from Croda (315 Cherry Lane New Castle Delaware 19720). Product code SPAN 40. EMPROVE^{®} Polyvinyl Alcohol 4-88 (PVA), USP (85-89% hydrolyzed, viscosity of 3.4-4.6 mPa s) was purchased from EMD Chemicals Inc. (480 South Democrat Road Gibbstown, NJ 08027). Product code 1.41350. Dulbecco's Phosphate Buffered Saline (DPBS), 1X, 0.0095 M (PO4), without calcium and magnesium, was purchased from BioWhittaker (8316 West Route 24 Mapleton, IL 61547). Part number 17-512Q. Emulsification was carried out using a Branson Digital Sonifier 250 with a 1/8" tapered tip titanium probe.

Solutions were prepared as follows:
**Solution 1:** A polymer and rapamycin solution was prepared by dissolving PS-PEG at 50 mg per mL and rapamycin at 8 mg per mL in dichloromethane. **Solution 2:** A polymer and rapamycin solution was prepared by dissolving PS-PMMA (Mw=30,000 Da) at 50 mg per mL and rapamycin at 8 mg per mL in dichloromethane. **Solution 3:** A lipid and rapamycin solution was prepared by dissolving DMPC at 50 mg per mL and rapamycin at 8 mg per mL in dichloromethane. **Solution 4:** A polymer and rapamycin solution was prepared by dissolving (P-123) at 50 mg per mL and rapamycin at 8 mg per mL in dichloromethane. **Solution 5:** Dichloromethane was sterile filtered using a 0.2 µm PTFE membrane syringe filter (VWR part number 28145-491). **Solution 6:** SPAN 40 was dissolved at 50 mg per mL in dichloromethane. **Solution 7:** PVA was dissolved at 62.5 mg per mL in 100 mM pH 8 phosphate buffer.

For sample 28, an O/W emulsion was prepared by combining Solution 1 (1.0 mL) and Solution 5 (0.05 mL) in a small glass pressure tube pre-chilled >4 minutes in an ice water bath. Next, Solution 7 (3.0 mL) was added, and the pressure tube was vortex mixed for ten seconds. The emulsion was then sonicated with the pressure tube immersed in an ice bath for 1 minute at 30% amplitude. The resulting nano-emulsion was then added to an open 50 mL beaker containing DPBS (15 mL), and a piece of aluminum foil was placed over the open beaker. A second emulsion was prepared using the same procedure, and added to the first emulsion in the same 50 mL beaker with a fresh aliquot of DPBS (15 mL). The beaker was left uncovered and the aluminum foil discarded. This was then stirred at room temperature for 2 hours to allow the dichloromethane to evaporate and for the nanocarriers to form. A portion of the nanocarriers were washed by transferring the nanocarrier suspension to a centrifuge tube and centrifuging at 75,600×g and 4 °C for 50 minutes, removing the supernatant, and re-suspending the pellet in DPBS. The wash procedure was repeated and then the pellet was re-suspended in DPBS to achieve a nanocarrier suspension having a nominal concentration of 10 mg per mL on a polymer basis. The nanocarrier formulation was filtered using a 0.22 µm PES membrane syringe filter (Millex part number SLGP033RS). The mass of the nanocarrier solution filter throughput was measured. The filtered nanocarrier solution was then stored at -20°C.

Sample 29 was prepared the same as for sample 28, using Solution 6 in place of Solution 5. Sample 30 was prepared the same as sample 28, using Solution 2 in place of Solution 1. Sample 31 was prepared the same as sample 30, using Solution 6 in place of Solution 5. Sample 32 was prepared the same as sample 28, using Solution 3 in place of Solution 1. Sample 33 was prepared the same as sample 32, using Solution 6 in place of Solution 5. Sample 34 was prepared the same as sample 28, using Solution 4 in place of Solution 1. Sample 35 was prepared the same as sample 34, using Solution 6 in place of Solution 5.

Nanocarrier size was determined by dynamic light scattering. The amount of rapamycin in the nanocarrier was determined by HPLC analysis. The total dry-nanocarrier mass per mL of suspension was determined by a gravimetric method. The filterability was evaluated by mass of the nanocarrier filtrate through the first 33 mm PES membrane 0.22 µm syringe filter.

Below the results show that, while not optimized, the inclusion of SPAN 40 in synthetic nanocarriers with non-polyester polymers can increase filterability of the synthetic nanocarriers in some embodiments.

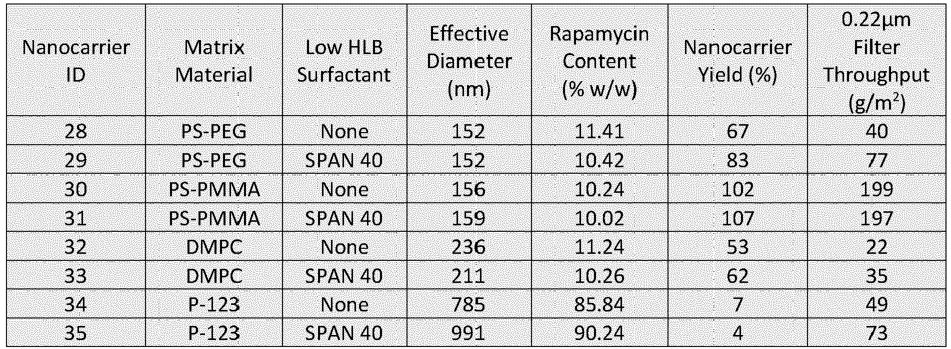

### Example 6 - SPAN 40 Greatly Increases Filterability of Synthetic Nanocarriers Comprising Polyester Polymers

### Materials and Methods

PLA (100 DL 4A), with an inherent viscosity of 0.41 dL/g was purchased from Evonik Industries AG (Rellinghauser Straβe 1-11, Essen Germany), product code 100 DL 4A. PLA-PEG-OMe block co-polymer with a methyl ether terminated PEG block of approximately 5,000 Da and an overall inherent viscosity of 0.50 DL/g was purchased from Evonik Industries AG (Rellinghauser Straβe 1-11, Essen Germany), product code 100 DL mPEG 5000 5CE. Rapamycin was purchased from Concord Biotech Limited (1482-1486 Trasad Road, Dholka 382225, Ahmedabad India), product code SIROLIMUS. EMPROVE^{®} Polyvinyl Alcohol 4-88 (PVA), USP (85-89% hydrolyzed, viscosity of 3.4-4.6 mPa·s) was purchased from EMD Chemicals Inc. (480 South Democrat Road Gibbstown, NJ 08027), product code 1.41350. Dulbecco's phosphate buffered saline 1X (DPBS) was purchased from Lonza (Muenchensteinerstrasse 38, CH-4002 Basel, Switzerland), product code 17-512Q. Sorbitan monopalmitate (SPAN 40), was purchased from Croda International (300-A Columbus Circle, Edison, NJ 08837), product code Span 40. PLGA (5050 DLG 2.5A), with approximately 54% by weight lactide and 46% by weight glycolide, and an inherent viscosity of 0.24 dL/g was purchased from Evonik Industries AG (Rellinghauser Straβe 1-11, Essen Germany), product code 5050 DLG2. 5A. PLGA (7525 DLG 4A), with approximately 73% by weight lactide and 27% by weight glycolide, and an inherent viscosity of 0.39 dL/g was purchased from Evonik Industries AG (Rellinghauser Straβe 1-11, Essen Germany), product code 7525 DLG 4A. Polycaprolactone (PCL), average Mw 14,000 Da and Mn of 10,000 Da, was purchased from Sigma-Aldrich (3050 Spruce St. St. Louis, MO 63103), product code 440752.

For samples 1, 3, 5 and 7, solutions were prepared as follows:
Solution 1: PLA-PEG-Ome at 50 mg per mL, Span 40 at 10 mg per mL and rapamycin at 32 mg per mL were dissolved in dichloromethane. Solution 2: 100 DL 4A was dissolved in dichloromethane at 150 mg per mL. Solution 3: 5050 DLG 2.5A was dissolved in dichloromethane at 150 mg per mL. Solution 4: 7525 DLG 4A was dissolved in dichloromethane at 150 mg per mL. Solution 5: PCL was dissolved in dichloromethane at 150 mg per mL. Solution 6: PVA was prepared at 75 mg per mL in 100 mM pH 8 phosphate buffer.

An O/W emulsion was prepared by transferring Solution 1 (0.5 mL), to a thick walled glass pressure tube. To this, lot 1 added Solution 2 (0.5 mL), lot 3 added Solution 3 (0.5 mL), lot 5 added 4 (0.5 mL), and lot 7 added Solution 5 (0.5 mL). The two solutions were then mixed by repeat pipetting. Next, Solution 6 (3.0 mL) was added, the tube was vortex mixed for 10 seconds, and was then emulsified by sonication at 30% amplitude for 1 minute with the pressure tube immersed in an ice water bath using a Branson Digital Sonifier 250. The emulsion was then added to a 50 mL beaker containing DPBS (30 mL). This was then stirred at room temperature for 2 hours to allow the dichloromethane to evaporate and for the nanocarriers to form. A portion of the nanocarriers was washed by transferring the nanocarrier suspension to a centrifuge tube and centrifuging at 75,600×g for 50 minutes, removing the supernatant, and re-suspended the pellet in DPBS. The wash procedure was repeated and then the pellet was re-suspended in DPBS to achieve a nanocarrier suspension having a nominal concentration of 10 mg/mL on a polymer basis. The nanocarrier suspension was then filtered using a 0.22 µm PES membrane syringe filter (Millipore part number SLGP033RB), and if necessary: 0.45 µm PES membrane syringe filter (PALL part number 4614), and/or a 1.2 µm PES membrane syringe filter (PALL part number 4656). The filtered nanocarrier suspension was then stored at -20°C.

Nanocarrier size was determined by dynamic light scattering. The amount of rapamycin in the nanocarrier was determined by HPLC analysis. Filterability was determined by comparing the weight of flow through of the first sterile 0.22 µm filter to the yield to determine the actual mass of nanocarriers that passed through prior to blocking the filter, or the total through the first and only filter. The total dry-nanocarrier mass per mL of suspension was determined by a gravimetric method.

For samples 2, 4, 6 and 8, solutions were prepared as follows:
Solution 1: A polymer and rapamycin mixture was prepared by dissolving PLA-PEG-Ome at 50 mg per mL, and rapamycin at 32 mg per mL in dichloromethane. Solution 2: 100 DL 4A was dissolved in dichloromethane at 150 mg per mL. Solution 3: 5050 DLG 2.5A was dissolved in dichloromethane at 150 mg per mL. Solution 4: 7525 DLG 4A was dissolved in dichloromethane at 150 mg per mL. Solution 5: PCL was dissolved in dichloromethane at 150 mg per mL. Solution 6: Polyvinyl alcohol was prepared at 75 mg per mL in 100 mM pH 8 phosphate buffer.

An O/W emulsion was prepared by transferring Solution 1 (0.5 mL), to a thick walled glass pressure tube. To this, lot 2 added Solution 2 (0.5 mL), lot 4 added Solution 3 (0.5 mL), lot 6 added 4 (0.5 mL), and lot 8 added Solution 5 (0.5 mL). The two solutions were then mixed by repeat pipetting. The addition of PVA solution, wash, filtration and storage are the same as above.

Nanocarrier size was evaluated the same as above.

The results show a significant increase in filterability of synthetic nanocarriers comprising polyester polymers with the inclusion of SPAN 40 in the synthetic nanocarriers.

| Lot number | Core polymer | Excipient | Size (nm) | Filter throughput (g NP/m2) | Rapa load (%) | NP yield (%) |
|---|---|---|---|---|---|---|
| 1 | 100 DL4A | SPAN 40 | 160 | >148 | 12.65 | 75 |
| 2 | 100 DL 4A | None | 197 | 17 | 10.88 | 71 |
| 3 | 5050 DLG 2.5A | SPAN 40 | 153 | >139 | 13.09 | 70 |
| 4 | 5050 DLG 2.5A | None | 188 | 59 | 13.40 | 64 |
| 5 | 7525 DLG 4A | SPAN 40 | 164 | >158 | 11.81 | 78 |
| 6 | 7525 DLG 4A | None | 196 | 28 | 11.64 | 73 |
| 7 | Polycaprolactone | SPAN 40 | 164 | 112 | 10.62 | 75 |
| 8 | Polycaprolactone | None | 173 | 52 | 10.29 | 78 |

### Example 7 - Synthetic Nanocarriers with Low HLB Surfactant and Significant RAPA Load

### Results in Durable Antigen-Specific Tolerance

### Materials and Methods

PLA with an inherent viscosity of 0.41 dL/g was purchased from Lakeshore Biomaterials (756 Tom Martin Drive, Birmingham, AL 35211), product code 100 DL 4A. PLA-PEG-OMe block co-polymer with a methyl ether terminated PEG block of approximately 5,000 Da and an overall inherent viscosity of 0.50 DL/g was purchased from Lakeshore Biomaterials (756 Tom Martin Drive, Birmingham, AL 35211), product code 100 DL mPEG 5000 5CE. Rapamycin was purchased from Concord Biotech Limited (1482-1486 Trasad Road, Dholka 382225, Ahmedabad India), product code SIROLIMUS. Sorbitan monopalmitate was purchased from Sigma-Aldrich (3050 Spruce St., St. Louis, MO 63103), product code 388920. EMPROVE^{®} Polyvinyl Alcohol (PVA) 4-88, USP (85-89% hydrolyzed, viscosity of 3.4-4.6 mPa·s) was purchased from EMD Chemicals Inc. (480 South Democrat Road Gibbstown, NJ 08027), product code 1.41350. Dulbecco's phosphate buffered saline 1X (DPBS) was purchased from Lonza (Muenchensteinerstrasse 38, CH-4002 Basel, Switzerland), product code 17-512Q.

Solutions were prepared as follows:
**Solution 1:** A polymer, rapamycin, and sorbitan monopalmitate mixture was prepared by dissolving PLA at 37.5 mg/mL, PLA-PEG-Ome at 12.5 mg/mL, rapamycin at 8 mg/mL, and sorbitan monopalmitate at 2.5 in dichloromethane. **Solution 2:** Polyvinyl alcohol was prepared at 50 mg/mL in 100 mM pH 8 phosphate buffer.

An O/W emulsion was prepared by combining Solution 1 (1.0 mL) and Solution 2 (3 mL) in a small glass pressure tube, vortex mixed for 10 seconds. The formulation was then homogenized by sonication at 30% amplitude for 1 minute. The emulsion was then added to an open beaker containing DPBS (30 mL). A second O/W emulsion was prepared using the same materials and method as above and then added to the same beaker containing the first emulsion and DPBS. The combined emulsion was then stirred at room temperature for 2 hours to allow the dichloromethane to evaporate and for the nanocarriers to form. A portion of the nanocarriers was washed by transferring the nanocarrier suspension to a centrifuge tube and centrifuging at 75,600×g and 4 °C for 50 minutes, removing the supernatant, and re-suspending the pellet in DPBS containing 0.25% w/v PVA. The wash procedure was repeated and then the pellet was re-suspended in DPBS containing 0.25% w/v PVA to achieve a nanocarrier suspension having a nominal concentration of 10 mg/mL on a polymer basis. The nanocarrier suspension was then filtered using a 0.22 µm PES membrane syringe filter (Millipore part number SLGP033RB). The filtered nanocarrier suspension was then stored at -20°C.

Nanocarrier size was determined by dynamic light scattering. The amount of rapamycin in the nanocarrier was determined by HPLC analysis. The total dry-nanocarrier mass per mL of suspension was determined by a gravimetric method.

| Effective Diameter (nm) | Rapamycin Content (% w/w) | Nanocarrier Conc (mg/mL) |
|---|---|---|
| 150 | 11.5 | 11.1 |

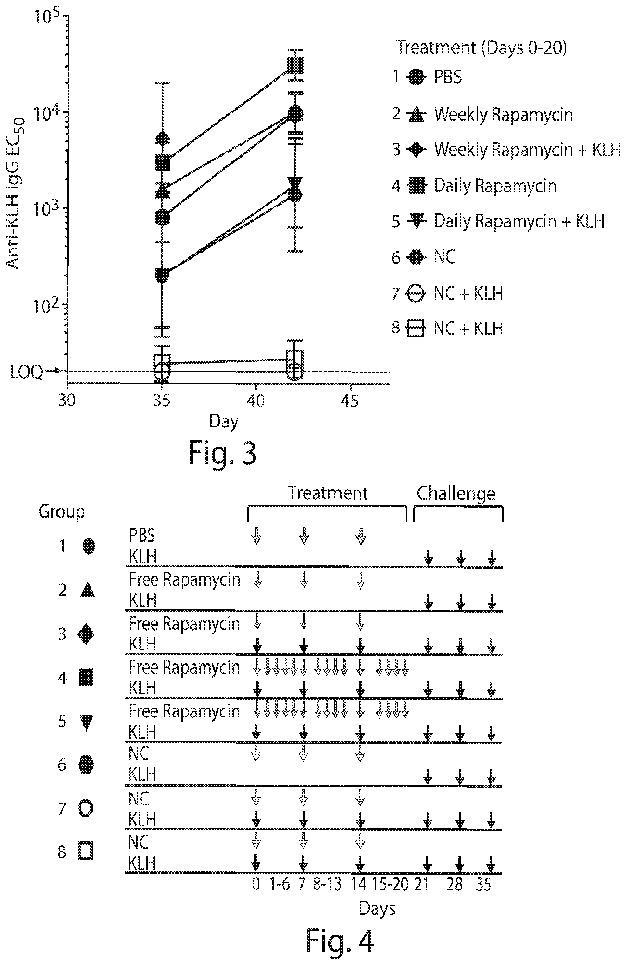

The ability of the synthetic nanocarriers, versus free rapamycin, to induce durable immune tolerance toward the model antigen KLH was evaluated. Groups of naive C57BL/6 mice (n=10 per group) were dosed intravenously on days 0, 7, and 14 with PBS (group 1), 50µg (~2mg/kg) free rapamycin alone or admixed with KLH (groups 2 and 3, respectively), or 50µg rapamycin encapsulated in synthetic nanocarriers alone (group 6) or admixed with KLH (groups 7 and 8) (**Fig. 3**). To determine the effects of chronic rapamycin administration group 4 received free rapamycin alone five times per week (50µg/day) from Day 0 to Day 20 or in combination with KLH administered once per week (group 5). All groups were subsequently challenged with 200 µg KLH on Days 21, 28 and 35. Sera were collected, and the anti-KLH antibody responses were measured on day 35 and 42 (after 2 and 3 injections, respectively). Efficacy was evaluated as the EC50 for anti-KLH antibody titer as determined by ELISA. **Fig. 4** illustrates the protocol.

Control PBS-treated mice developed high levels of anti-KLH antibodies on days 35 and 42, after 2 and 3 challenge injections of KLH, respectively. Mice treated with free rapamycin (either weekly or daily) in the absence of KLH developed similar levels of anti-KLH antibodies as the PBS-treated group. Mice treated with synthetic nanocarriers alone or with daily free rapamycin and KLH showed a delayed response compared to the PBS control group, but the titers boosted with each challenge with KLH. These results indicate that treatment with synthetic nanocarriers alone does not induce chronic immunosuppression, and that KLH administered with daily free rapamycin, even at 5 times the total weekly dose of rapamycin as administered in synthetic nanocarriers, does not induce durable immunological tolerance.

By contrast, mice treated with synthetic nanocarriers+KLH (groups 7 and 8), that comprised a significant amount of rapamycin, developed little or no detectable anti-KLH antibodies, even after receiving three weekly post-treatment KLH challenges (for a total of 6 KLH injections), indicating durable immune tolerance. Both lots of synthetic nanocarriers were similarly effective. All groups except for those treated with synthetic nanocarrier+KLH developed anaphylactic reactions by day 42. These results indicate that tolerization to KLH induced by treatment with synthetic nanocarrier comprising a significant amount of rapamycin and KLH prevented the development of hypersensitivity reactions.

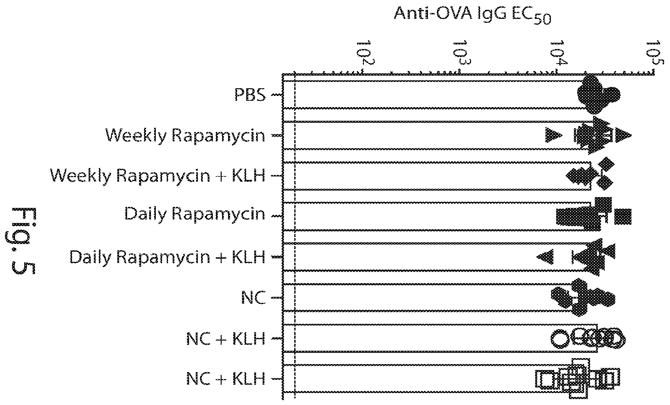

To evaluate the antigen specificity of the tolerance to KLH, all animals were challenged with OVA+CpG s.c. (35µg+20µg) in the hind limb on Days 49 and 56. **Fig. 5** shows that all animals developed similar levels of titers against OVA demonstrating that concomitant administration of the antigen with synthetic nanocarrier can yield immunological tolerance and that synthetic nanocarrier treatment does not induce chronic immunosuppression. These results demonstrate that nanocarrier-encapsulated, rather than free rapamycin, (when present at a significant amount, induced durable and antigen-specific immune tolerance when concomitantly administered with a target antigen.

### Example 8 - SPAN 40 Increases Filterability of Rapalogs, Rapamycin and Everolimus

### Materials and Methods

PLA with an inherent viscosity of 0.41 dL/g was purchased from Evonik Industries AG (Rellinghauser Straße 1-11, Essen Germany), product code 100 DL 4A. PLA-PEG-OMe block co-polymer with a methyl ether terminated PEG block of approximately 5,000 Da and an overall inherent viscosity of 0.50 DL/g was purchased from Evonik Industries AG (Rellinghauser Straße 1-11, Essen Germany), product code 100 DL mPEG 5000 5CE. Rapamycin was purchased from Concord Biotech Limited (1482-1486 Trasad Road, Dholka 382225, Ahmedabad India), product code SIROLIMUS. Everolimus was purchased from LC Laboratories (165 New Boston St # T, Woburn, MA 01801). Product code E-4040. Temsirolimus was purchased from LC Laboratories (165 New Boston Street Woburn, MA 01801). Part number T-8040. Deforolimus was purchased from MedChem Express (11 Deer Park Drive, Suite 102D Monmouth Junction, NJ 08852). Product code HY-50908. EMPROVE^{®} Polyvinyl Alcohol 4-88, USP (85-89% hydrolyzed, viscosity of 3.4-4.6 mPa s) was purchased from EMD Chemicals Inc. (480 South Democrat Road Gibbstown, NJ 08027), product code 1.41350. Dulbecco's phosphate buffered saline 1X (DPBS) was purchased from Lonza (Muenchensteinerstrasse 38, CH-4002 Basel, Switzerland), product code 17-512Q. Sorbitan monopalmitate was purchased from Croda International (300-A Columbus Circle, Edison, NJ 08837), product code SPAN 40.

Solutions were prepared as follows. **Solution 1:** A polymer and rapamycin mixture was prepared by dissolving PLA at 150 mg/mL and PLA-PEG-Ome at 50 mg/mL. **Solution 2:** A rapamycin solution was prepared at 100 mg/mL in dichloromethane. **Solution 3:** An everolimus solution was prepared at 100 mg/mL in dichloromethane. **Solution 4:** A temsirolimus solution was prepared at 100 mg/mL in dichloromethane. **Solution 5:** A deforolimus solution was prepared at 100 mg/mL in dichloromethane. **Solution 6:** A sorbitan monopalmitate solution was prepared by dissolving SPAN 40 at 50 mg/mL in dichloromethane. **Solution 7:** Polyvinyl alcohol was prepared at 75 mg/mL in 100 mM pH 8 phosphate buffer.

O/W emulsions were prepared by adding Solution 1 (0.5mL), to a thick walled pressure tube. For lots 1, 3, 5, and 7, this was combined with Solution 6 (0.1 mL), and dichloromethane (0.28 mL). Lot 1 then combined these with Solution 2 (0.12 mL), Lot 3 with Solution 3 (0.12 mL), Lot 5 with Solution 4 (0.12 mL), and Lot 7 with Solution 4 (0.12 ,mL). In a similar manner, Lots 2, 4, 6, and 8 were combined with dichloromethane (0.38 mL), and then Lot 2 combined with Solution 2 (0.12 mL), Lot 4 with Solution 3 (0.12 mL), Lot 6 with Solution 4 (0.12 mL), and Lot 8 with Solution 5 (0.12 mL). For each individual lot the total volume of the organic phase was therefore 1 mL. The combined organic phase solutions were mixed by repeat pipetting. Next, Solution 7 (3.0 mL) was added, the pressure tube was vortex mixed for 10 seconds, and was then sonicated at 30% amplitude for 1 minute with the pressure tube immersed in an ice water bath using a Branson Digital Sonifier 250. The emulsion was then added to a 50 mL beaker containing DPBS (30 mL). This was then stirred at room temperature for 2 hours to allow the dichloromethane to evaporate rapidly for the nanocarriers to form. A portion of the nanocarriers was washed by transferring the nanocarrier suspension to a centrifuge tube and centrifuging at 75,600×g and 4 °C for 50 minutes, removing the supernatant, and re-suspended the pellet in DPBS containing 0.25% w/v PVA. The wash procedure was repeated and then the pellet was re-suspended in DPBS containing 0.25% w/v PVA to achieve a nanocarrier suspension having a nominal concentration of 10 mg/mL on a polymer basis. The nanocarrier suspension was then filtered using a 0.22 µm PES membrane syringe filter (Millipore part number SLGP033RB). The filtered nanocarrier suspension was then stored at -20°C.

The results show that the incorporation of SPAN 40 in synthetic nanocarriers increased the filterability of the rapalogs, rapamycin and everolimus.

| Lot number | Rapalog | Low HLB Surfactant | Calculated Filter Throughput (g NP/m2) | Size (nm) | Yield (%) | Rapalog load (%) |
|---|---|---|---|---|---|---|
| 1 | Rapamycin | SPAN 40 | >117 | 163 | 60 | 10.41 |
| 2 | Rapamycin | None | 21 | 189 | 58 | 11.38 |
| 3 | Everolimus | SPAN 40 | >130 | 165 | 66 | 13.60 |
| 4 | Everolimus | None | 23 | 190 | 57 | 12.03 |
| 5 | Temsirolimus | SPAN 40 | 31 | 163 | 73 | 8.73 |
| 6 | Temsirolimus | None | 46 | 178 | 62 | 9.57 |
| 7 | Deforolimus | SPAN 40 | >119 | 135 | 61 | 7.51 |
| 8 | Deforolimus | None | >120 | 139 | 60 | 7.53 |

### Example 9 - Shows the Effects of the Amounts of the Components on Rapamycin Load and Synthetic Nanocarrier Filterability

### Materials and Methods

PLA-PEG-OMe block co-polymer with a methyl ether terminated PEG block of approximately 5,000 Da and an overall inherent viscosity of 0.50 DL/g was purchased from Evonik Industries (Rellinghauser Straße 1-11 45128 Essen, Germany), product code 100 DL mPEG 5000 5CE. PLA with an inherent viscosity of 0.41 dL/g was purchased from Evonik Industries (Rellinghauser Straße 1-11 45128 Essen Germany), product code 100 DL 4A. Rapamycin was purchased from Concord Biotech Limited, 1482-1486 Trasad Road, Dholka 382225, Ahmedabad India. Product code SIROLIMUS. Sorbitan monopalmitate was purchased from Croda (315 Cherry Lane New Castle Delaware 19720), product code SPAN 40. Dichloromethane was purchased from Spectrum (14422 S San Pedro Gardena CA, 90248-2027). Part number M1266. EMPROVE^{®} Polyvinyl Alcohol 4-88, (PVA), USP (85-89% hydrolyzed, viscosity of 3.4-4.6 mPa·s) was purchased from EMD Chemicals Inc. (480 South Democrat Road Gibbstown, NJ 08027), product code 1.41350. Dulbecco's Phosphate Buffered Saline (DPBS), 1X, 0.0095 M (PO4), without calcium and magnesium, was purchased from BioWhittaker (8316 West Route 24 Mapleton, IL 61547), part number #12001, product code Lonza DPBS. Emulsification was carried out using a Branson Digital Sonifier 250 with a 1/8" tapered tip titanium probe.

Solutions were prepared as follows:
**Polymer Solution:** A polymer mixture was prepared by dissolving PLA-PEG-OMe (100 DL mPEG 5000 5CE) and PLA (100 DL 4A) at the indicated mg per mL in dichloromethane at a 1:3 ratio of PLA-PEG to PLA. **Rapamycin Solution:** Rapamycin was dissolved at the indicated mg per 1 mL in dichloromethane. **SPAN 40 Solution:** Sorbitan monopalmitate (SPAN 40) was dissolved at the indicated mg per mL in dichloromethane. **CH2Cl2 Solution:** Dichloromethane (CH2Cl2), was sterile filtered using a 0.2µm PTFE membrane syringe filter (VWR part number 28145-491). **PVA Solution:** A polyvinyl alcohol solution was prepared by dissolving polyvinyl alcohol (EMPROVE^{®} Polyvinyl Alcohol 4-88) at the indicated mg per 1 mL in 100 mM pH 8 phosphate buffer. **DPBS PVA Solution:** A polyvinyl alcohol and Dulbecco's phosphate buffered saline, 1X, 0.0095 M (PO4) mixture was prepared by dissolving polyvinyl alcohol (EMPROVE^{®} Polyvinyl Alcohol 4-88) at 2.5 mg per 1 mL in Dulbecco's phosphate buffered saline, 1X, 0.0095 M (PO4) (Lonza DPBS).

An O/W emulsion was prepared by combining the Polymer Solution, Rapamycin Solution, SPAN 40 Solution and/or CH2Cl2 Solution (Total volume 1-2 mL) in a thick walled glass pressure tube. The solution was mixed by repeat pipetting. Next, PVA Solution (3 to 6 mL) was added (ether as a single emulsion with 1 mL organic phase and 3 mL aqueous PVA Solution, or as two single emulsions prepared one after the other). The formulation was vortex mixed for ten seconds, and then sonicated with the pressure tube immersed in an ice bath for 1 minute at 30% amplitude. The emulsion was then added to an open 50 mL beaker containing Lonza DPBS (30 mL). This was then stirred at room temperature for 2 hours to allow the dichloromethane to evaporate and for the nanocarriers to form. A portion of the nanocarriers were washed by transferring the nanocarrier suspension to a centrifuge tube and centrifuging at 75,600×g and 4 °C for 50 minutes, removing the supernatant, and re-suspending the pellet in DPBS PVA Solution. The wash procedure was repeated and then the pellet was re-suspended in DPBS PVA Solution to achieve a nanocarrier suspension having a nominal concentration of 10 mg per mL on a polymer basis. The nanocarrier formulation was filtered using a 0.22µm PES membrane syringe filter (Millex part number SLGP033RS). The mass of the nanocarrier solution filter throughput was measured. The filtered nanocarrier solution was then stored at -20°C.

Filterability is given as g/m² of filter membrane surface area, of measured nanocarrier passing through one 33 mm PES membrane 0.22 µm syringe filter from Millipore, part number SLGP033RB.

The results show the amount of various components in a number of synthetic nanocarriers that can result in initial sterile filterable synthetic nanocarriers with an amount of rapamycin that is expected to be efficacious *in vivo.*

| Lot# | Polymer (mg per mL) | SPAN 40 (mg per mL) | Rapamycin (mg per mL) | PVA (mg per mL) | Size (nm) | Filterability (g NP/m²) | Yield | wt% HLB/Rapa | wt% HLB/Polymer |
|---|---|---|---|---|---|---|---|---|---|
| 1^{a} | 50 | 0 | 8 | 62.5 | 135 | 52 | 70.7 | 0.00 | 0.00 |
| 2^{a} | 50 | 0.1 | 8 | 62.5 | 135 | 26 | 68.6 | 1.23 | 0.20 |
| 3^{a} | 50 | 0.25 | 8 | 62.5 | 148 | 27 | 70.9 | 3.03 | 0.50 |
| 4^{a} | 50 | 0.5 | 8 | 62.5 | 166 | 146 | 73.2 | 5.88 | 0.99 |
| 5^{a} | 50 | 1 | 8 | 62.5 | 147 | 151 | 75.7 | 11.11 | 1.96 |
| 6^{a} | 50 | 1.5 | 8 | 62.5 | 161 | 146 | 72.2 | 15.79 | 2.91 |
| 7^{a} | 50 | 2.5 | 8 | 62.5 | 149 | 176 | 85.0 | 23.81 | 4.76 |
| 8^{a} | 50 | 2.5 | 8 | 50 | 182 | 209 | 103.5 | 23.81 | 4.76 |
| 9^{a} | 50 | 2.5 | 8 | 75 | 132 | 155 | 76.7 | 23.81 | 4.76 |
| 10^{a} | 50 | 3 | 8 | 62.5 | 143 | 140 | 69.4 | 27.27 | 5.66 |
| 11^{a} | 62.5 | 3 | 8 | 62.5 | 151 | 205 | 80.9 | 27.27 | 4.58 |
| 12^{a} | 37.5 | 3 | 8 | 62.5 | 139 | 203 | 60.9 | 27.27 | 7.41 |
| 13^{a} | 50 | 4.5 | 8 | 62.5 | 149 | 149 | 73.6 | 36.00 | 8.26 |
| 14^{a} | 50 | 5 | 6.66 | 50 | 148 | 193 | 94.4 | 42.88 | 9.09 |
| 15^{a} | 50 | 5 | 8.33 | 50 | 176 | 176 | 86.2 | 37.48 | 9.09 |
| 16^{a} | 50 | 10 | 8 | 50 | 173 | 38 | 66.1 | 55.56 | 16.67 |
| 17 | 100 | 10 | 11.32 | 75 | 153 | 178 | 88.2 | 46.90 | 909 |
| 18 | 100 | 10 | 14.16 | 75 | 160 | 200 | 98.9 | 41.39 | 909 |
| 19 | 100 | 10 | 17 | 75 | 177 | 182 | 101.0 | 3704 | 909 |
| 20 | 100 | 7.5 | 24 | 75 | 188 | 125 | 70.4 | 23.81 | 6.98 |
| 21 | 75 | 11.25 | 30 | 75 | 197 | 17 | 82.5 | 27.27 | 13.04 |
| 22 | 100 | 15 | 32 | 75 | 201 | 17 | 108.1 | 31.91 | 13.04 |
| 23 | 100 | 15 | 40 | 75 | 217 | 9 | 82.6 | 27.27 | 13.04 |
| 24 | 100 | 15 | 40 | 75 | 193 | 14 | 116.5 | 27.27 | 13.04 |

| | | | | | | | | | |
|---|---|---|---|---|---|---|---|---|---|
| ^{a}These formulations were prepared with 2 mL organic phase, 6 mL PVA Solution. | | | | | | | | | |

## Claims

1. A composition comprising synthetic nanocarriers comprising:
a hydrophobic carrier material,
a rapalog, and
a non-ionic surfactant with a hydrophilic-lipophilic balance (HLB) value less than or equal to 10;
wherein the amount of non-ionic surfactant with HLB value less than or equal to 10 is ≥ 0.01 but ≤ 20 weight% non-ionic surfactant with a HLB value less than or equal to 10/weight hydrophobic carrier material,
wherein, at least 80%, at least 90%, or at least 95% of the synthetic nanocarriers, based on the total number of synthetic nanocarriers, have a minimum dimension or maximum dimension that falls within 5%, 10%, or 20% of the average diameter or average dimension of the synthetic nanocarriers; and
preferably wherein the composition is initially sterile filterable through a 0.22 µm filter.

2. A method for producing synthetic nanocarriers comprising a non-ionic surfactant with HLB value less than or equal to 10 and a rapalog, comprising:
obtaining or providing a hydrophobic carrier material,
obtaining or providing a non-ionic surfactant with HLB value less than or equal to 10,
obtaining or providing a rapalog, and
combining the hydrophobic carrier material, the non-ionic surfactant with HLB value less than or equal to 10 and the rapalog to form synthetic nanocarriers,
wherein the amount of non-ionic surfactant with HLB value less than or equal to 10 in the synthetic nanocarriers is ≥ 0.01 but ≤ 20 weight% non-ionic surfactant with a HLB value less than or equal to 10/weight hydrophobic carrier material, and
wherein, at least 80%, at least 90%, or at least 95% of the synthetic nanocarriers, based on the total number of synthetic nanocarriers, have a minimum dimension or maximum dimension that falls within 5%, 10%, or 20% of the average diameter or average dimension of the synthetic nanocarriers.

3. The method of claim 2, further comprising:
dissolving the hydrophobic carrier material, non-ionic surfactant with HLB value less than or equal to 10 and rapalog in a solvent;
obtaining or providing another surfactant;
forming a first and then second O/W emulsion with the dissolved hydrophobic carrier material, non-ionic surfactant with HLB value less than or equal to 10 and rapalog, and other surfactant;
mixing the first and second O/W emulsions; and
allowing the solvent to evaporate, preferably wherein the solvent is dichloromethane, ethyl acetate, chloroform, or propylene carbonate.

4. The composition of claim 1 or the method of claims 2 or 3, wherein:
(i) the non-ionic surfactant with HLB value less than or equal to 10 is a non-ionic surfactant with HLB value less than 10;
(ii) the non-ionic surfactant with HLB value less than or equal to 10 is a non-ionic surfactant with HLB value less than 9;
(iii) the non-ionic surfactant with HLB value less than or equal to 10 is a non-ionic surfactant with HLB value less than 8;
(iv) the non-ionic surfactant with HLB value less than or equal to 10 is a non-ionic surfactant with HLB value less than 7;
(v) the non-ionic surfactant with HLB value less than or equal to 10 is a non-ionic surfactant with HLB value less than 6; or
(vi) the non-ionic surfactant with HLB value less than or equal to 10 is a non-ionic surfactant with HLB value less than 5.

5. The composition of claims 1 or 4, or the method of any of claims 2 to 4, wherein:
(i) the non-ionic surfactant with HLB value less than or equal to 10 comprises a sorbitan ester, fatty alcohol, fatty acid ester, ethoxylated fatty alcohol, poloxamer or a fatty acid;
(ii) the non-ionic surfactant with HLB value less than or equal to 10 comprises a sorbitan ester, fatty alcohol, fatty acid ester, ethoxylated fatty alcohol, poloxamer or a fatty acid, and wherein the non-ionic surfactant with HLB value less than or equal to 10 comprises SPAN 40, SPAN 20, oleyl alcohol, stearyl alcohol, isopropyl palmitate, glycerol monostearate, BRIJ 52, BRIJ 93, Pluronic P-123, Pluronic L-31, palmitic acid, dodecanoic acid, glyceryl tripalmitate or glyceryl trilinoleate; or
(iii) the non-ionic surfactant with HLB value less than or equal to 10 is SPAN 40.

6. The composition of any of claims 1, 4 or 5, or the method of any of claims 2 to 5, wherein the non-ionic surfactant with HLB value less than or equal to 10 is encapsulated in the synthetic nanocarriers, present on the surface of the synthetic nanocarriers, or both.

7. The composition of any of claims 1 or 4 to 6, or the method of any of claims 2 to 6, wherein:
(i) the amount of non-ionic surfactant with HLB value less than or equal to 10 is ≥ 0.1 but ≤ 15 weight% non-ionic surfactant with a HLB value less than or equal to 10/weight hydrophobic carrier material;
(ii) the amount of non-ionic surfactant with HLB value less than or equal to 10 is ≥ 1 but ≤ 13 weight% non-ionic surfactant with a HLB value less than or equal to 10/weight hydrophobic carrier material; or
(iii) the amount of non-ionic surfactant with HLB value less than or equal to 10 is ≥ 1 but ≤ 9 weight% non-ionic surfactant with a HLB value less than or equal to 10/weight hydrophobic carrier material.

8. The composition of any of claims 1 or 4 to 7, or the method of any of claims 2 to 7, wherein:
(i) the hydrophobic carrier material comprises one or more hydrophobic polymers or lipids;
(ii) the hydrophobic carrier material comprises one or more hydrophobic polymers, and wherein the one or more hydrophobic polymers comprise a polyester, preferably wherein the polyester comprises PLA, PLG, PLGA or polycaprolactone; or
(iii) the hydrophobic carrier material comprises one or more hydrophobic polymers, and wherein the one or more hydrophobic polymers comprise a polyester, preferably wherein the polyester comprises PLA, PLG, PLGA or polycaprolactone, and wherein the hydrophobic carrier material comprises or further comprises PLA-PEG, PLGA-PEG or PCL-PEG.

9. The composition of any of claims 1 or 4 to 8, or the method of any of claims 2 to 8, wherein:
(i) the amount of hydrophobic carrier material in the synthetic nanocarriers is 5-95 weight% hydrophobic carrier material/total solids; or
(ii) the amount of hydrophobic carrier material in the synthetic nanocarriers is 60-95 weight% hydrophobic carrier material/total solids.

10. The composition of any of claims 1 or 4 to 9, or the method of any of claims 2 to 9, wherein:
(i) the amount of rapalog is ≥ 6 but ≤ 50 weight% rapalog/weight hydrophobic carrier material;
(ii) the amount of rapalog is ≥ 7 but ≤ 30 weight% rapalog/weight hydrophobic carrier material; or
(iii) the amount of rapalog is ≥ 8 but ≤ 24 weight% rapalog/weight hydrophobic carrier material.

11. The composition of any of claims 1 or 4 to 10, or the method of any of claims 2 to 10, wherein the rapalog is encapsulated in the synthetic nanocarriers and/or wherein the rapalog is rapamycin.

12. The composition of any of claims 1 or 4 to 11, wherein:
(i) the composition further comprises an antigen; or
(ii) the composition further comprises an antigen, wherein the antigen is admixed with the synthetic nanocarriers in the composition.

13. The composition of any of claims 1 or 4 to 12, wherein the mean of a particle size distribution obtained using dynamic light scattering of the synthetic nanocarriers is a diameter greater than 120nm, preferably wherein the diameter is greater than 150nm.

14. The composition of any of claims 1 or 4 to 13, wherein the composition further comprises a pharmaceutically acceptable carrier.

15. A kit comprising: the composition of any of claims 1 or 4 to 14.
